(19) Europäisches Patentamt / European Patent Office / Office européen des brevets

(11) **EP 4 631 535 A1**

(12) **EUROPEAN PATENT APPLICATION**

(43) Date of publication:
**15.10.2025   Bulletin 2025/42**

(21) Application number: **24206692.6**

(22) Date of filing: **15.10.2024**

(51) International Patent Classification (IPC):
**A61L 27/12** *(2006.01)*    **A61L 27/36** *(2006.01)*
**A61L 27/46** *(2006.01)*    **A61L 27/54** *(2006.01)*

(52) Cooperative Patent Classification (CPC):
(C-Sets available)
**A61L 27/46; A61L 27/12; A61L 27/365;**
**A61L 27/54;** A61L 2300/254; A61L 2300/30;
A61L 2300/414; A61L 2430/02           (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **11.04.2024   EP 24169595**

(71) Applicant: **novaxomx GmbH**
**65933 Frankfurt am Main (DE)**

(72) Inventor: **The designation of the inventor has not
yet been filed**

(74) Representative: **Fuchs Patentanwälte
Partnerschaft mbB
Tower 185
Friedrich-Ebert-Anlage 35-37
60327 Frankfurt am Main (DE)**

Remarks:
The complete document including Reference
Table(s) and the Sequence Listing(s) can be
downloaded from the EPO website

(54)    **COMPOUNDS FOR USE AS BONE REPLACEMENT AND/OR BONE REBUILDING MATERIAL**

(57)    Disclosed are platelet-derived extracellular vesicles (EVs), and composite materials comprising them, for use as bone replacement material and/or as bone rebuilding material. Also, methods of production, medical uses and kits are disclosed.

FIGURE 1

(52) Cooperative Patent Classification (CPC): (Cont.)

C-Sets
**A61L 27/46, C08L 75/04;**
**A61L 27/46, C08L 89/06**

**Description**

Disclosed are platelet-derived extracellular vesicles (EVs) and composite materials comprising them for use as bone replacement material and/or as bone rebuilding material. Also, methods of production, medical uses and kits are disclosed.

Background of the Disclosure

Bone degeneration diseases, such as osteoporosis and osteoarthritis, play significant roles in society due to their prevalence, impact on healthcare systems, and effects on quality of life. They contribute significantly to the healthcare burden in society. They are associated with a wide range of medical complications, including fractures, chronic pain, and mobility issues, all of which require medical attention, treatment, and management.

Also, in the dental sector bone degeneration diseases such as for example periodontal disease, osteonecrosis of the jaw (ONJ), jawbone osteoporosis, periapical lesions, osteomyelitis, and Paget's disease of bone also inflicting dental development are known.

These diseases impose a considerable economic burden on society due to healthcare costs, including hospitalizations, medications, surgeries, and rehabilitation services. Additionally, bone degeneration diseases can lead to disability, decreased productivity, and loss of income for affected individuals and their caregivers.

As populations age worldwide, the prevalence of bone degeneration diseases is expected to increase, posing a significant public health challenge. Efforts to prevent and manage these diseases, including education, early detection, and access to appropriate healthcare services, are crucial to mitigating their impact on public health.

Bone degeneration diseases can have a profound impact on the quality of life of affected individuals. Chronic pain, loss of mobility, and functional limitations can impair daily activities, independence, and overall well-being. These conditions also affect mental health, leading to increased rates of depression, anxiety, and social isolation.

The prevalence and impact of bone degeneration diseases have spurred research efforts to better understand their underlying mechanisms, risk factors, and potential treatments. Advances in medical science and technology, including novel medications, surgical techniques, and rehabilitation strategies, offer hope for improved outcomes and quality of life for affected individuals.

There are some therapies aimed at fostering bone regrowth and addressing bone degeneration diseases. These include bone-building medications with bisphosphonates, such as alendronate and risedronate, which are commonly prescribed to treat osteoporosis by slowing down bone resorption and promoting bone formation. Teriparatide and abaloparatide are medications that may be used to stimulate bone formation and to treat severe osteoporosis or to increase bone mass before fracture repair. Selective estrogen receptor modulators (SERMs) like raloxifene can help prevent bone loss in postmenopausal women by mimicking the effects of estrogen on bone tissue.

Other therapies include the use of nutritional supplements, such as calcium and vitamin D supplements, which are often recommended to maintain bone health and prevent osteoporosis. Adequate intake of these nutrients is essential for bone mineralization and remodeling.

Another treatment can be the change of lifestyle, such as regular weight-bearing exercises, such as walking, jogging, or weightlifting, which can help strengthen bones and reduce the risk of osteoporosis. Also smoking cessation and limiting alcohol consumption can help maintain bone health, as these habits are associated with increased bone loss. Physical therapy and rehabilitation programs can help improve strength, flexibility, and mobility in individuals with bone degeneration diseases, thereby reducing the risk of falls and fractures.

If pharmacological or physical therapies fail, surgical Interventions may be applied. In cases of severe bone loss or fracture, surgical interventions such as bone grafting, joint replacement, or fracture fixation may be necessary to restore bone structure and function.

Growth factors and cytokines, such as bone morphogenetic proteins (BMPs) or platelet-rich plasma (PRP), may also be used to stimulate bone regeneration and repair in certain conditions, such as bone defects or non-union fractures. Emerging therapies in gene editing and gene therapy also hold promise for treating bone degeneration diseases by targeting specific genes involved in bone formation and remodeling. For example, stem cell-based approaches, including mesenchymal stem cell (MSC) therapy and induced pluripotent stem cell (iPSC) technology, are being investigated for their potential to regenerate bone tissue and repair skeletal defects.

However, all of the existing therapies are also associated with potential disadvantages and risks.

Many medications used to treat bone degeneration diseases, such as bisphosphonates and anabolic agents, have side effects. These include gastrointestinal issues, musculoskeletal pain, and in rare cases, osteonecrosis of the jaw or atypical femoral fractures. Further, not all patients respond equally well to medications and other therapies for bone degeneration diseases. Some individuals may not experience significant improvement in bone density or fracture risk reduction, despite treatment.

Some medications, particularly bisphosphonates, have been associated with long-term safety concerns, including an increased risk of fractures after prolonged use, as well as potential adverse effects on bone quality. Some treatments for bone degeneration diseases, such as bisphosphonates, may also require long-term or lifelong use to maintain their benefits. This can lead to dependency on medications and ongoing healthcare expenses.

Also, emerging therapies, such as gene therapy and stem cell therapy, have promising potential for treating bone degeneration diseases, but their long-term safety and efficacy are still being studied. There may be unknown risks associated with these treatments that require further investigation.

On the other hand, surgical interventions for bone degeneration diseases, such as joint replacement or fracture repair, carry inherent risks, including infection, blood clots, and anesthesia complications. Recovery from surgery may also require a prolonged period of rehabilitation and physical therapy.

Many of the therapies mentioned, including medications, surgical procedures, and biologic treatments, can be costly. This may pose a barrier to access for some patients, particularly those without adequate insurance coverage or financial resources.

Here we present new compounds and uses for the prevention of bone loss and the stimulation of bone recovery, which are easy to employ, act locally and show superior effects as compared to existing prior art technologies.

Brief Summary of the Disclosure

In a first aspect, the present disclosure relates to a platelet-derived extracellular vesicle for use as bone replacement and/or bone rebuilding material.

In a second aspect, the present disclosure relates to a composite material for use as bone replacement and/or bone rebuilding material, characterized in comprising the platelet-derived extracellular vesicle as disclosed, further comprising at least one inorganic componentand at least one organic component.

In a third aspect, the present disclosure relates to methods for producing the disclosed composite material.

In a fourth aspect, the present disclosure relates to the platelet-derived extracellular vesicle according to the first aspect and/or the composite material according to the second aspect for use in medicine, such as in the treatment of a disease or condition selected from the group consisting of broken bones, bone fractures, bone loss, bone degradation, dental soft tissue degradation, dental bone diseases, periondontitis, alveolar bone resorption, dental implant placement, jaw bone infections associated with bone loss, osteomyelitis, osteonecrosis of the jaw (ONJ), osteoporosis, osteopenia, Paget's disease of bone, osteomalacia, rickets, hyperparathyroidism when associated with bone reduction, osteogenesis imperfecta (brittle bone disease), multiple myeloma when associated with bone reduction, metastatic bone disease (bone metastases), avascular necrosis (osteonecrosis), fibrous dysplasia, Gaucher's disease, hyperthyroidism when associated with bone reduction, renal osteodystrophy, anorexia nervosa when associated with bone reduction, Cushing's syndrome, rheumatoid arthritis when associated with bone reduction, psoriatic arthritis when associated with bone reduction, sarcoidosis when associated with bone reduction, systemic lupus erythematosus (SLE), thalassemia, hypophosphatasia when associated with bone reduction, Camurati-Engelmann disease, sickle cell disease when associated with bone reduction, chronic kidney disease when associated with bone reduction, marfan syndrome, Ehlers-Danlos syndrome (subtypes which are when associated with bone reduction), steroid-induced osteoporosis (resulting from long-term use of corticosteroids), inflammatory bowel disease (e.g., Crohn's disease, ulcerative colitis, which affect bone density), HIV/AIDS when associated with bone reduction, hemochromatosis when associated with bone reduction, post-polio syndrome affecting bones, diseases of dysfunction of bone metabolism, stimulation of osteoblasts, reduction of osteoclasts, migration/proliferation of (gingival) fibroblasts, and mastocytosis when associated with bone reduction, or combinations thereof.

In a fifth aspect, the present disclosure relates to a kit comprising the platelet-derived extracellular vesicle according to the first aspect and/or the composite material according to the second aspect, and further comprising at least one item selected from the group consisting of an applicator, a measuring cup, a mixing tool, a spatula, an instruction manual, a package leaflet, and a syringe, as well as any combination thereof.

Brief Description of the Figures

If not noted otherwise, asterisks in the figures relate to the p-value of significance of the measurements: $* = p < 0.05$, $** = p < 0.01$, $*** \ p < 0.001$, $**** \ p < 0.0001$; # is also used in some figures to show the significance of a value in comparison to the related controls: $\# = p < 0.05$.

**Figure 1** - Bar diagram of relative activity of osteoblasts. The differentiation of human osteoblasts was measured on a cover glass. Osteoblasts stimulated with EVs according to the present disclosure show a significant ($p < 0.01$) higher ratio in osteoblast expression compared to the untreated control.

**Figure 2** - Representative microphotographs of TRAP-staining of human osteoclasts (red) stimulated with RANKL.

Osteoclasts treated with EVs show significant less activity of osteoclasts under RANKL stimulation.

TRAP, or Tartrate-Resistant Acid Phosphatase, is an enzyme that plays a significant role in the function of osteoclasts, the cells responsible for bone resorption. It is used as a marker to identify osteoclasts and to indicate their activity.

RANKL stands for "Receptor Activator of Nuclear Factor Kappa-B Ligand." It plays a critical role in bone metabolism, particularly in regulating bone resorption. RANKL binds to its receptor RANK on the surface of osteoclasts and their precursor cells, leading to the formation, activation, and survival of osteoclasts. Osteoclasts are the cells responsible for bone tissue breakdown. The RANKL-RANK pathway is crucial for maintaining the balance between bone resorption and bone formation. Overexpression of RANKL or increased activity of this pathway can lead to enhanced bone breakdown and is associated with various bone diseases, including osteoporosis, rheumatoid arthritis, and bone metastases in cancer.

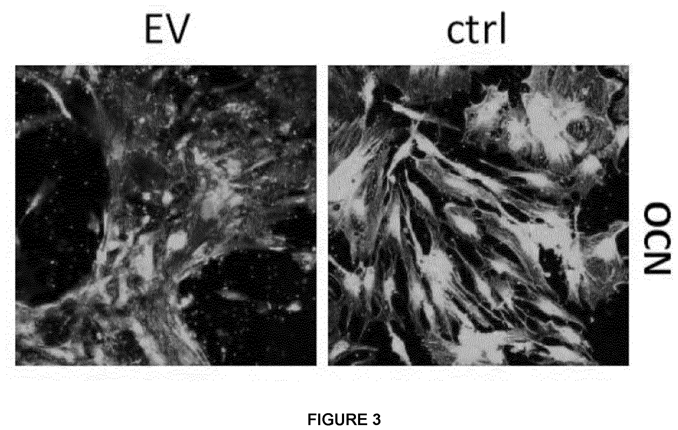

**Figure 3** - Immuno-fluorescence staining of osteocalcin as a marker of human osteoblast differentiation on cover glass. Osteoblasts with EV show compact positive osteocalcin in differentiated osteoblasts.

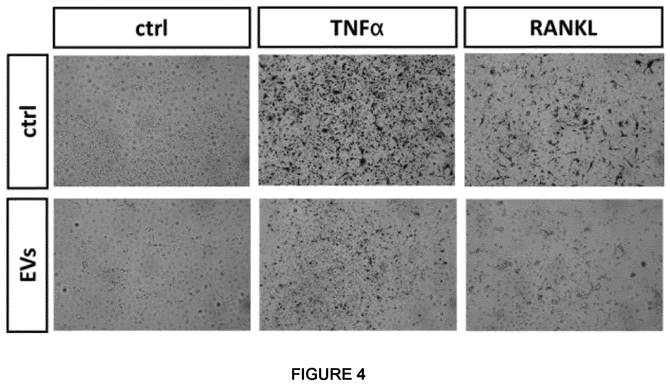

**Figure 4** - Representative microphotographs of TRAP-staining of murine osteoclasts. Osteoclasts treated with EVs show suppressed osteoclast differentiation even under stimulation with TNFalpha and RANKL.

TNF-alpha (Tumor Necrosis Factor-alpha) is a cytokine, a type of protein involved in cellular communication. It plays a crucial role in the immune system by regulating immune cells, inflammation, and the body's response to infection, injury, and diseases, including autoimmune diseases. TNF-alpha is involved in the inflammatory process. TNF-alpha can promote the differentiation and activation of osteoclasts. It does so, in part, by enhancing the expression of RANK (Receptor Activator of Nuclear Factor $\kappa$B) on the surface of osteoclast precursor cells and by increasing the production of RANKL (RANK Ligand) by osteoblasts and stromal cells. The interaction between RANKL and RANK is crucial for osteoclast differentiation and activation. By promoting this interaction, TNF-alpha indirectly stimulates osteoclastogenesis-the formation of new osteoclasts. TNF-alpha and RANKL work synergistically to promote osteoclastogenesis. While RANKL is a primary driver of osteoclast differentiation, TNF-alpha can enhance this effect, leading to increased bone resorption. This synergy is particularly relevant in conditions characterized by inflammation and excessive bone loss, such as rheumatoid arthritis. In diseases with an inflammatory component, such as rheumatoid arthritis and periodontal disease, TNF-alpha is significantly involved in bone destruction. The elevated levels of TNF-alpha found in these conditions contribute to the increased activity and number of osteoclasts, leading to pathological bone resorption.

Thus, the data shows that EVs of the present disclosure suppress osteoclast differentiation even under TNFalpha and RANKL, thereby reducing bone degradation and bone resorption.

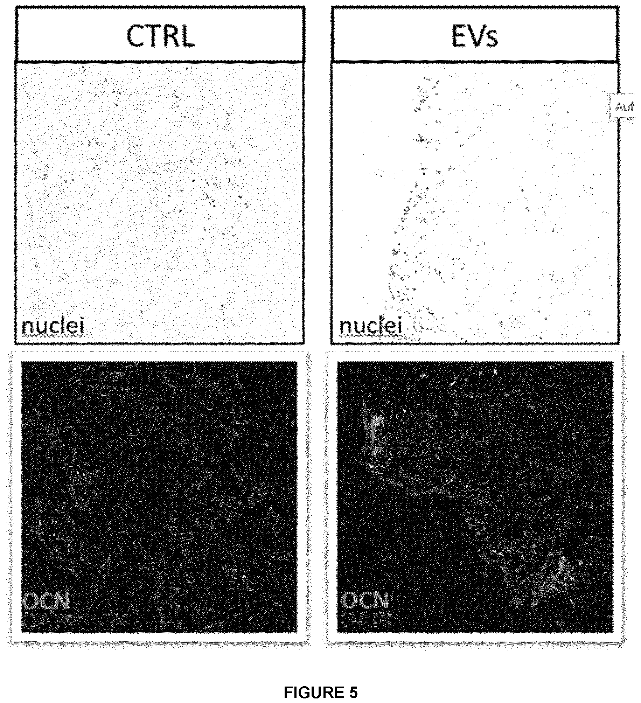

Figure 5 - Figure 6 the upper two images show nuclei of murine osteoblast cultured into $\beta$-TCP-collagen scaffold (left) and with EVs (right). The lower two images show DAPI staining of murine osteoblast cultured into $\beta$-TCP-collagen scaffold merged wirth immuno-fluorescence staining of osteocalcin; (osteoblast marker osteocalcin antibody (green), and nuclei were stained with DAPI (blue)). EV containing group shows significant higher migration and proliferation of osteocalcin positive osteoblasts into $\beta$-TCP-collagen scaffold. This is a significant indicator for the improvement of bone regeneration under the EVs of the present disclosure.

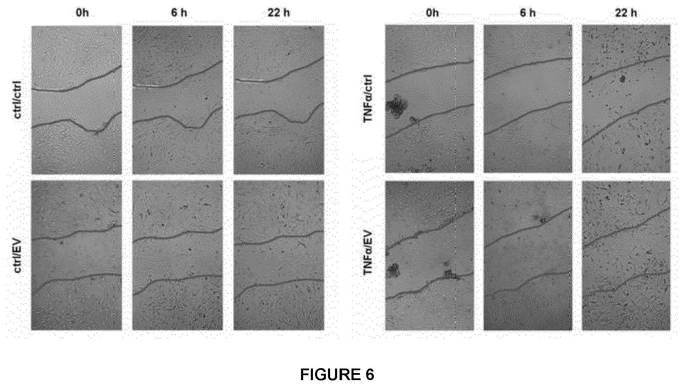

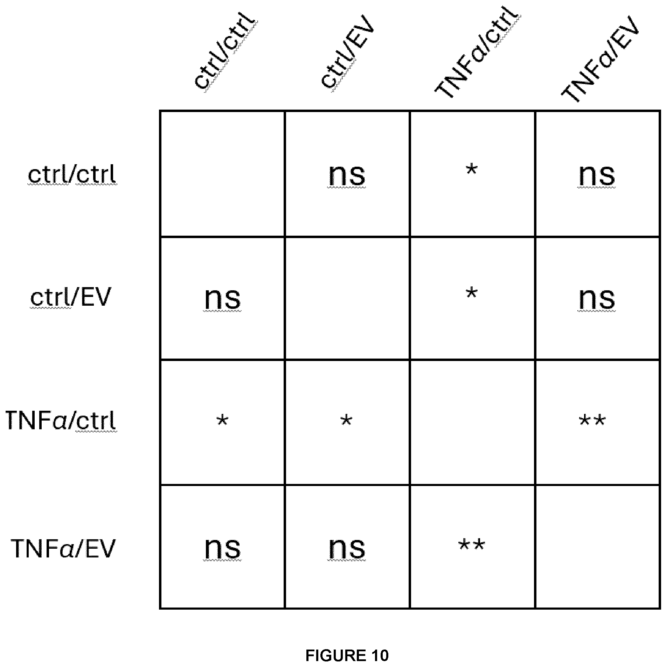

**Figure 6** - In-vitro scratch assay of murine fibroblasts. The closing of the wound gap was measured after 6 and 22h. The comparison of ctrl fibroblasts vs. EV containing fibroblasts samples show increased cell migration and regrowth of fibroblasts with EVs as compared to control (left). Further, also in assays in which the cells were additional stimulated with TNFalpha (right) the samples comprising the EVs of the present disclosure show increased cell migration. This shows that the EVs of the present disclosure improve wound healing even under inflammatory conditions. (cf. also Figure 10).

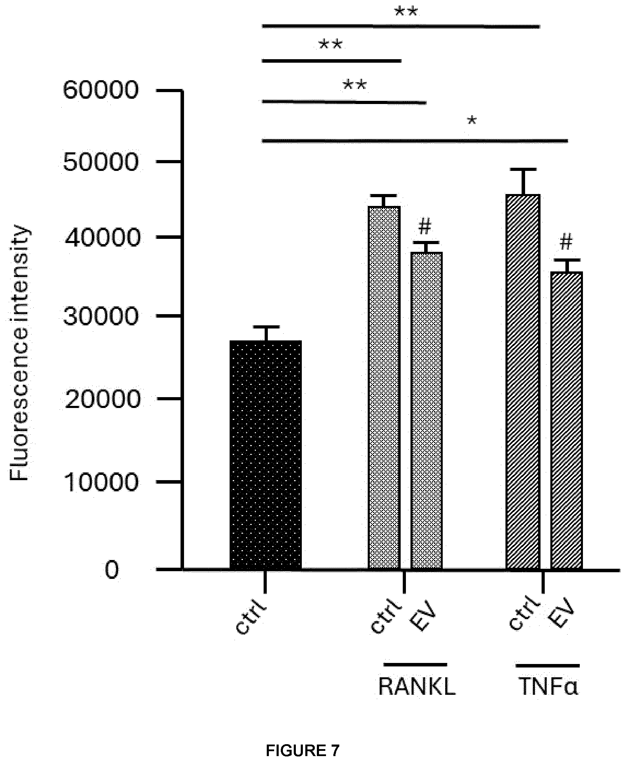

**Figure 7** - Bar diagram of fluorescence intensity of human osteoclast under RANKL and TNFalpha stimulation. First bar: unstimulated control cells. EV-treated cells (bar two vs. three) result in significant reduction of osteoclast-activity under RANKL stimulation (p < 0.05).And results in significant decrease in osteoclast-activity under TNF$\alpha$ stimulation (bar four vs. five) (p < 0.05). This again shows that cells containing EVs according to the present disclosure suppress osteoclast differentiation by inhibiting bone resorption.

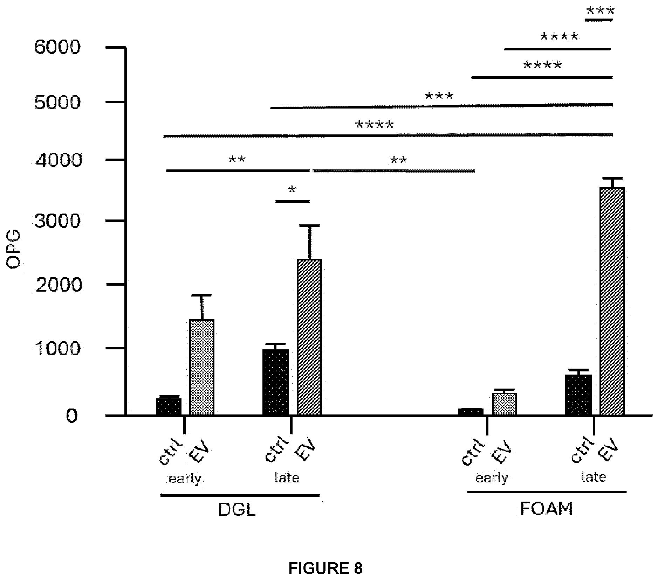

**Figure 8** - Quantative expression of Osteoprotegerin in co-culture with $\beta$-TCP-collagen scaffold under EV stimulation. EV group shows significant increase of OPG Expression in cover glass and significant higher OPG Expression in Foam. Osteoprotegerin (OPG) is another key player in this system. OPG acts as a "decoy" receptor for RANKL; by binding to RANKL, it prevents RANKL from interacting with RANK, thereby reducing the formation and activity of osteoclasts. Thus, OPG serves as a natural inhibitor of bone breakdown. This example shows that not only cells with EVs according to the disclosure show reduced bone breakdown or improved bone protection, respectively (left bars). Further, the right bars show that the composite material according to the disclosure shows surprisingly even better protection against bone breakdown. All results are significant.

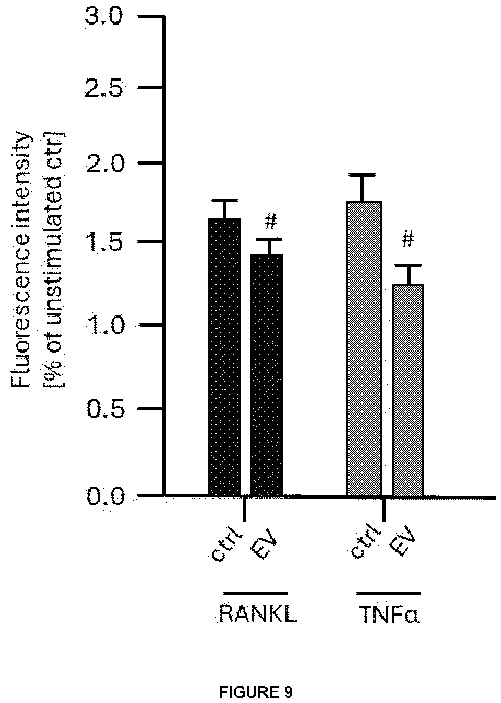

**Figure 9** - Fluorescence intensity human osteoclast in % of unstimulated cells. Compared are again cells under RANKL or TNFalpha stimulation without and with EVs. EV containing sample shows suppression of osteoclast differentiation under RANKL and TNFalpha compared to control. Again, the results were significant (p < 0.05).

**Figure 10** - Comparison chart of the different scratch assays. Compared were control-cells (fibroblasts) with and without TNFalpha-stimulation vs. sample cells with EV (also with and without TNFalpha-stimulation). "ns" means "not significant". Significant differences (p < 0.05) are depicted with one asterisk, highly significant differences are depicted by two asterisks (p < 0.01). Compare also example 4.1 for details. (cf. also Figure 6).

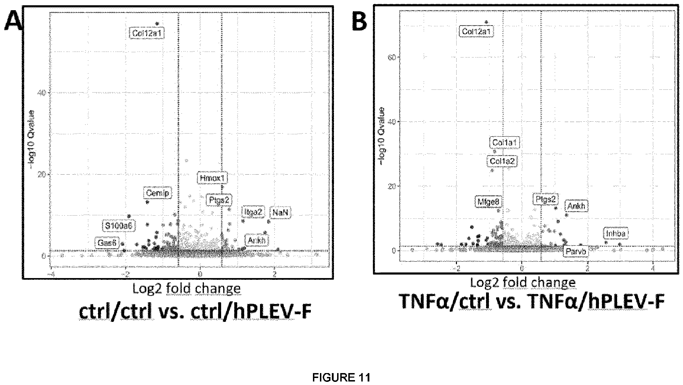

**Figure 11** - Treatment with hPLEV-F leads to selectively altered phosphorylation patterns in osteoblasts. Figures A and B depict Volcano plots as log2 fold change of protein expression plotted against log10 p-value of each feature. (A) comparison of untriggered (ctrl) and (B) TNF$\alpha$-stimulated cells with (hPLEV-F) and without (ctrl) hPLEV-treatment. Downstream analysis revealed up- and downregulated kinases under control conditions and under inflammatory TNF$\alpha$-stimulation.

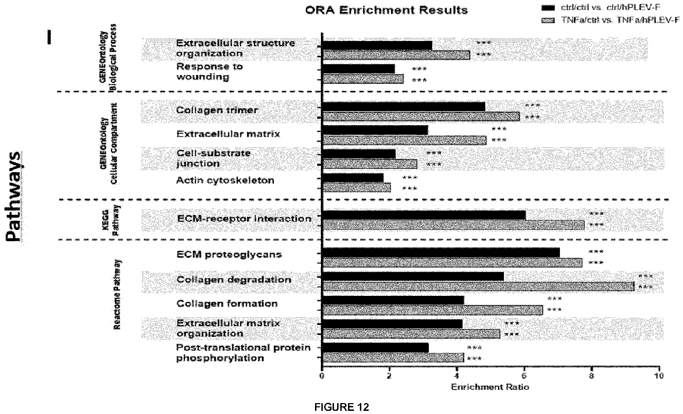

**Figure 12** - ORA Enrichment Results: Bone remodelling dependent pathways derived from over representation analysis (ORA) hPLEV-F treatment (black bars) and TNF$\alpha$ and hPLEV-F treatment (grey bars). ***p < 0.001.

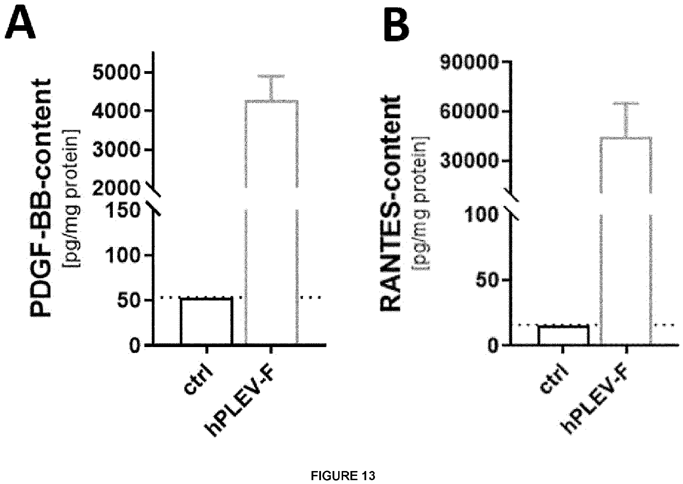

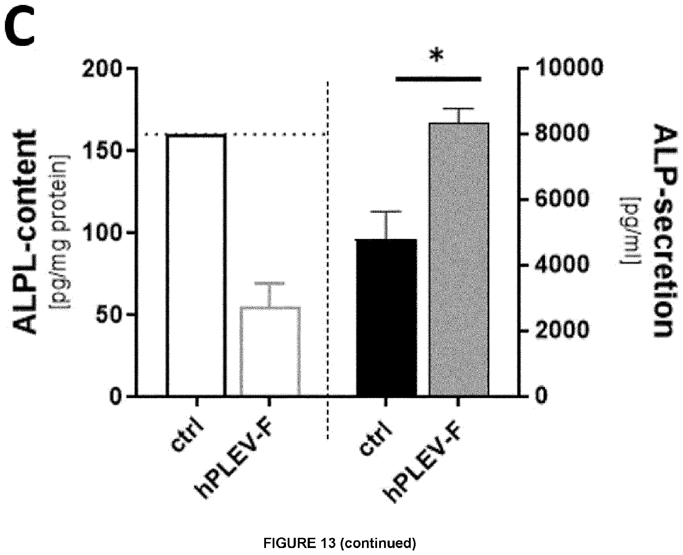

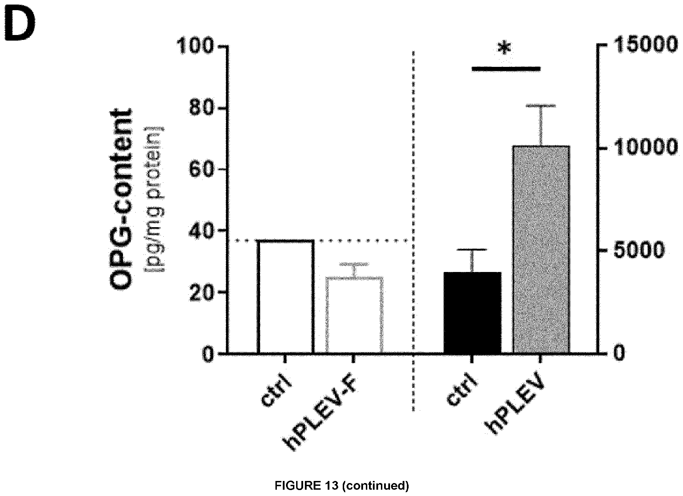

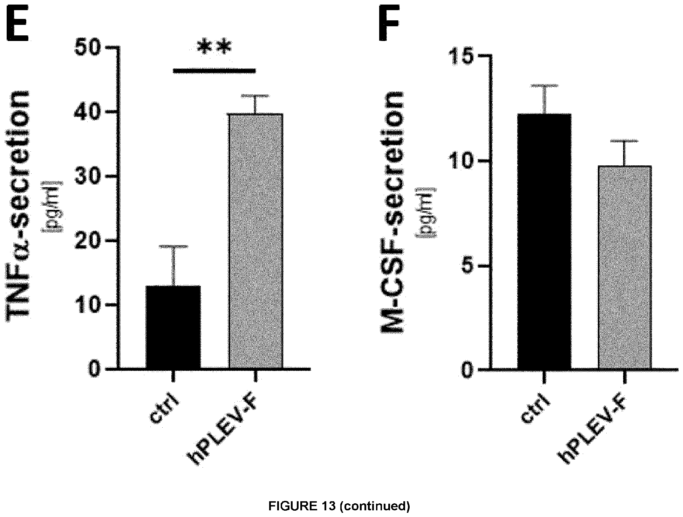

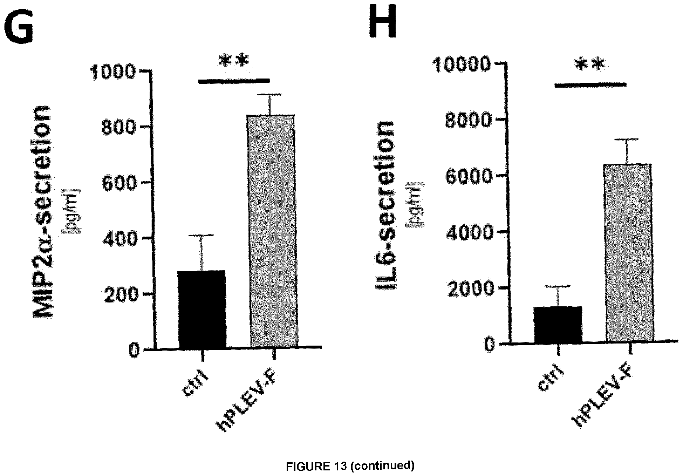

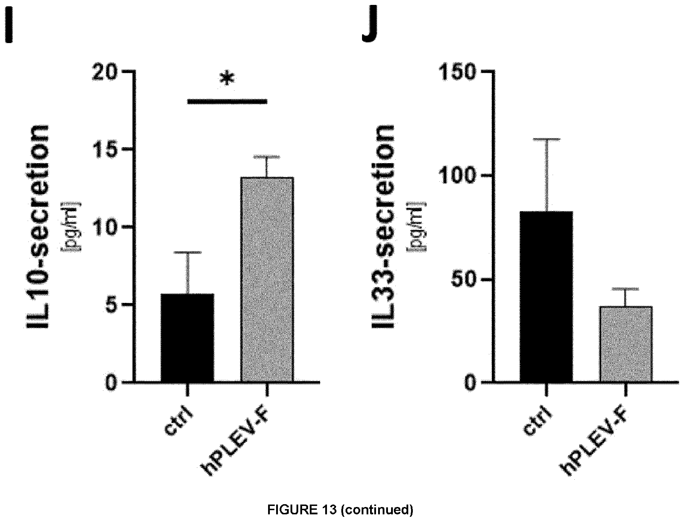

**Figure 13** - Treatment with hPLEV-F ameliorates osteoblastic proliferation and differentiation. Murine osteoblast (mOB) proliferation activity was analysed in primary cultures after 3 days of proliferation using a crystal violet staining. mOBs were treated with hPLEV-Fs and inflammatory trigger (TNF$\alpha$) and respective controls. (A-C) The hPLEV-Fs were analysed for content of PDGF-BB (A), RANTES (B) and (C) ALPL (open bars) in comparison to a control (C-right). (D) ELISA-measurements of averaged OPG within the hPLEV-Fs (open bars) and osteoblastic OPG-secretion (filled bars) into the cellular supernatant. The hPLEV-F-dependent changes in secretion of (E) TNF$\alpha$, (F) M-CSF, (G) MIP2$\alpha$, (H) IL6, (I) IL10 and (J) IL33 from 7-days-predifferentiated mOB. *p $\leq$ 0.05; **p < 0.01. Dotted lines indicating level of control. Statistical analyses: **A,B,C,D** (open bars): no statistical analyses, n of 1 for control.

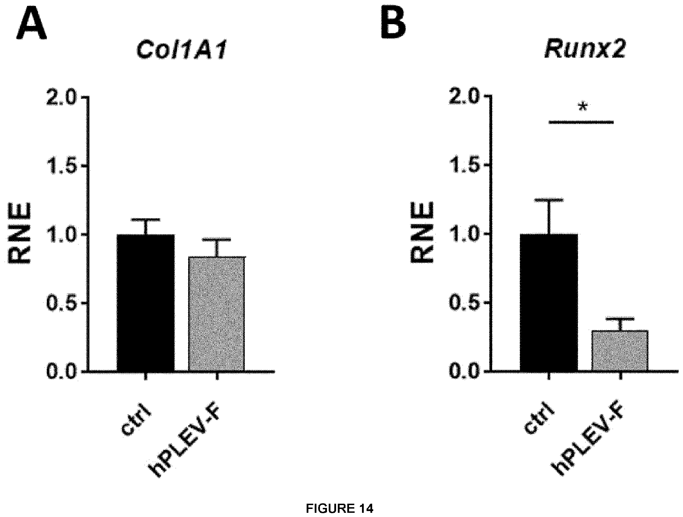

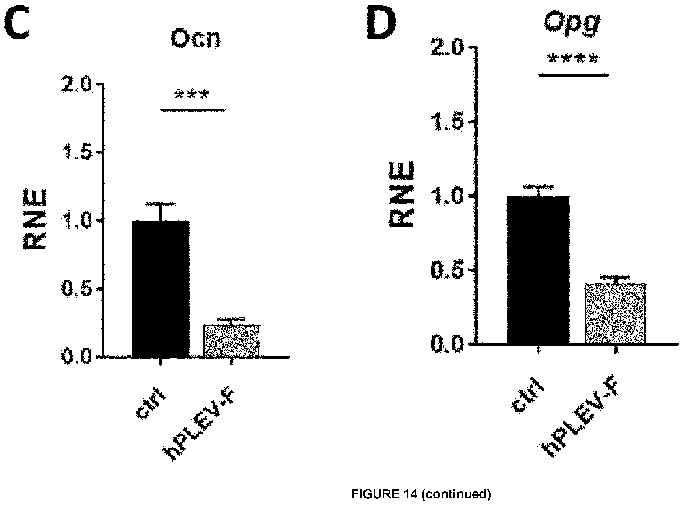

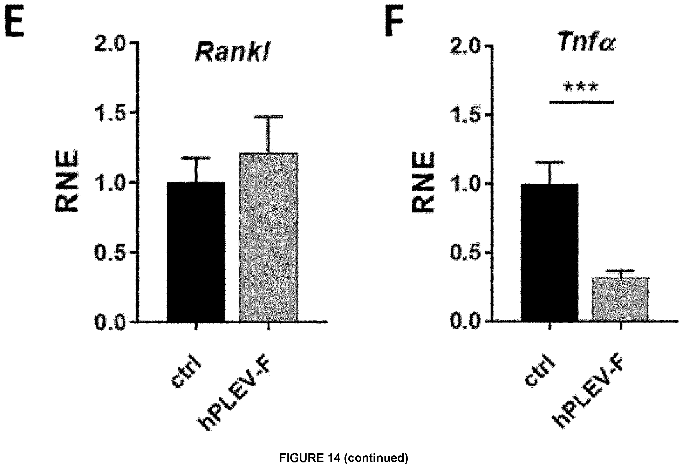

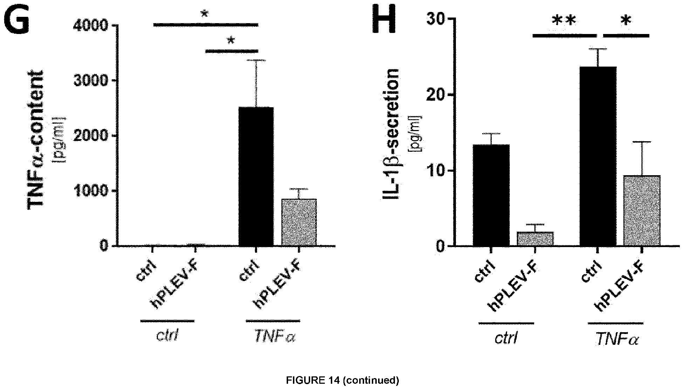

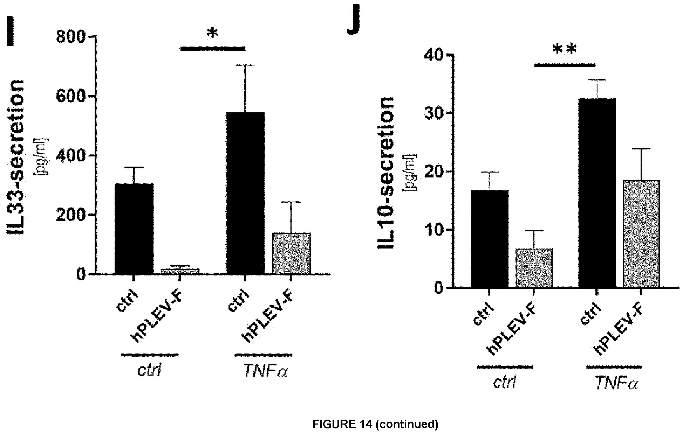

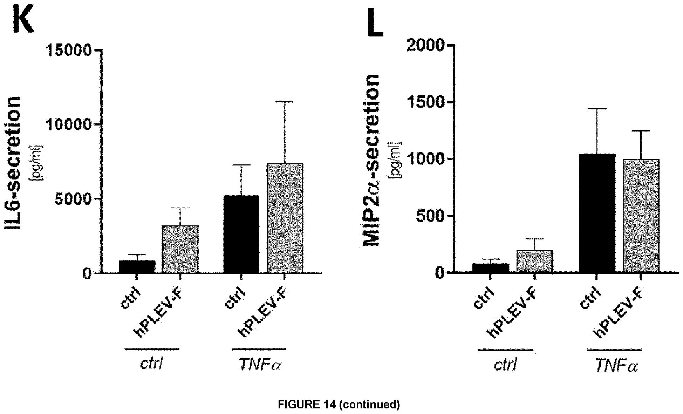

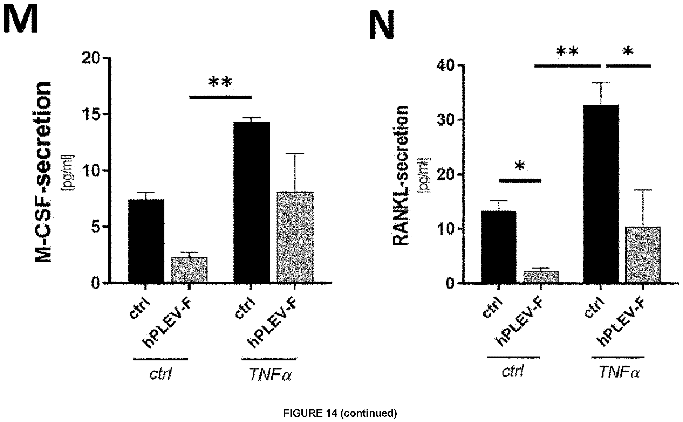

**Figure 14** - Gene expression analysis (Figure 14 A-G): Gene expression differences under hPLEV-F-treatment in 6 day differentiated mOB are shown for (A) Col1A1, (B) Runx2, (C) Ocn, (D)Opg, (E) Rankl and (F) Tnf$\alpha$. The overall TNF$\alpha$ concentration in supernatants taken after 7 days of treatment with differentiation medium, hPLEV-F and an inflammatory TNF$\alpha$ trigger (ctrl/ctrl, ctrl/hPLEV-F, TNF$\alpha$/ctrl, TNF$\alpha$/hPLEV-F) showed almost no basal content of TNF$\alpha$ and slightly higher content under inflammatory stimulus, which was reduced by addition of hPLEV-F (G). Secretion analysis (Figure 14 H-N): Secretion of IL-1$\beta$ (H), IL-33 (I), IL-10 (J), IL-6 (K), MIP2$\alpha$ (L), M-CSF (M) and RANKL (N) was analysed in the respective cultures. Statistical analyses: A-F Student's t-test. G-N two-way ANOVA with Tukey's post hoc tests; *p $\leq$ 0.05; **p < 0.01; ***p < 0.001; ****p<0.0001.

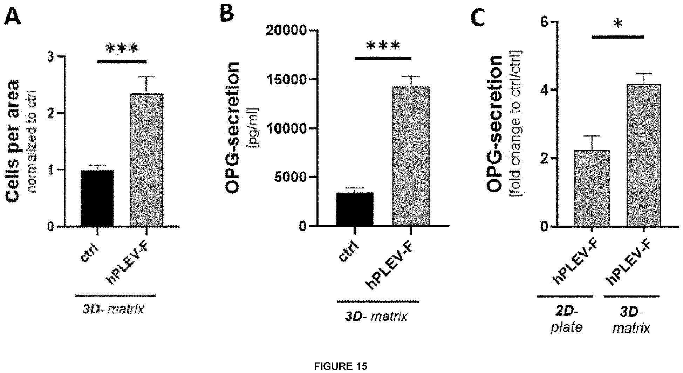

**Figure 15** - hPLEV-F-treatment improves differentiation of matrix-seeded cells and improves bone-protecting properties. (A) Microscopic pictures were quantified for cellular abundance per area and given as fold change of control cells (ctrl). (B, C) Cell culture supernatant was analyzed for OPG-secretion (B) from cells grown in the $\beta$-TCP-collagen composite of the disclosure ('3D') in pg/ml and (C) as fold change of respective control in classical cell culture conditions ('2D') compared to 3D on the $\beta$-TCP-collagen composite of the disclosure. Statistical analyses: Student's ttest; *p $\leq$ 0.05; ***p < 0.001.

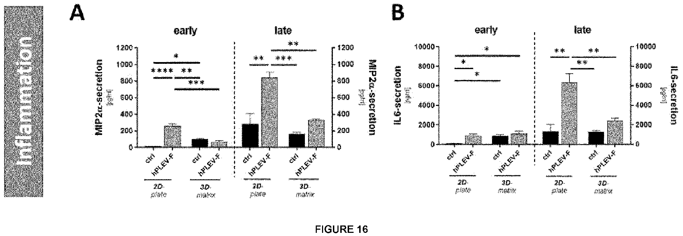

**Figure 16** - Treatment with hPLEV-F balances pro- and anti-inflammatory signalling. Changes in secretion at the early (4 days) and late (13 days) phase of differentiation were measured in supernatants, showing the difference between a 2D-plate and a 3D-matrix in hPLEV-F-treated mOB. MIP2$\alpha$ (A) and IL-6 (B) were determined as inflammatory markers. Statistical analyses: two-way ANOVA with Tukey's post hoc tests. *p $\leq$ 0.05; **p < 0.01; ***p < 0.001; ****p<0.0001.

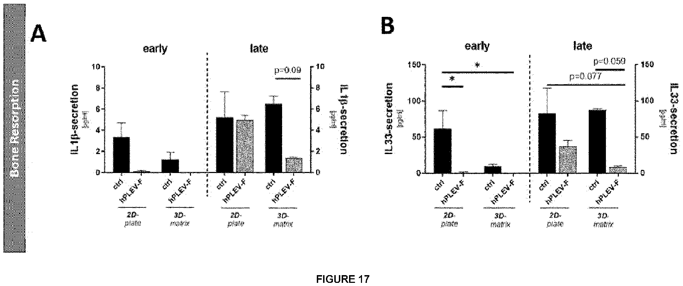

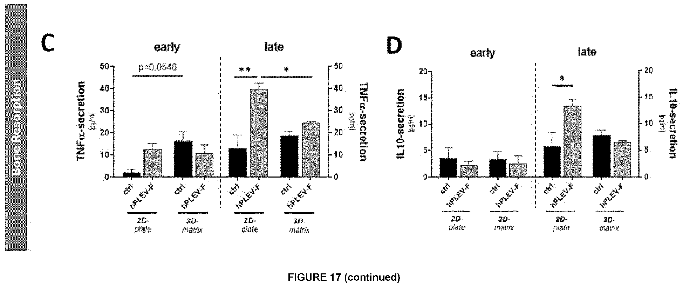

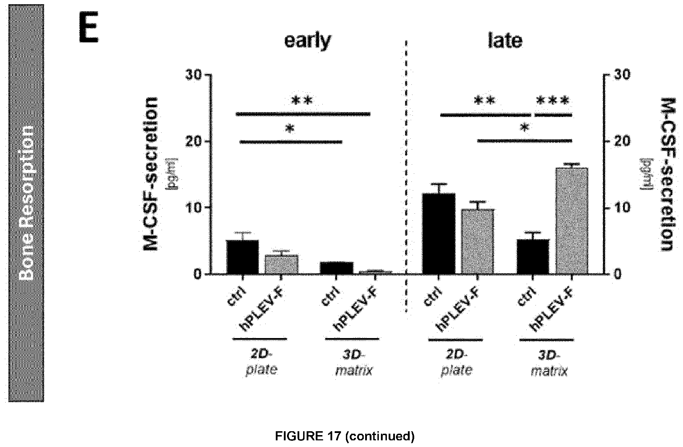

**Figure 17** - Treatment with hPLEV-F regulates bone metabolism. Changes in secretion at the early (4 days) and late (13 days) phase of differentiation were measured in supernatants, showing the difference between a 2D-plate and a 3D-matrix in hPLEV-F-treated mOB. IL-1$\beta$ (A), TNF$\alpha$ (B), IL-33 (C), IL-10 (D) and M-CSF (E) were analyzed as bone metabolism regulators. Statistical analyses: two-way ANOVA with Tukey's post hoc tests. *p $\leq$ 0.05; **p < 0.01; ***p < 0.001; ****p<0.0001.

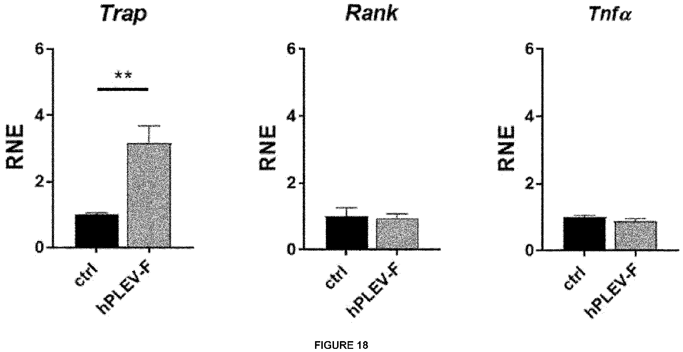

**Figure 18** - hPLEV-F reduces TNF$\alpha$- and RANKL-mediated osteoclast differentiation and resorption activity. Murine bone marrow cells were stimulated (A) with M-CSF supplemented medium in presence and absence of hPLEV-F. (A) After 48h of treatment, cells were investigated for Trap-, Rank- and Tnf$\alpha$-expression depicted as fold change of unstimulated cells. Cells were stained for TRAP after 5 days of TNF$\alpha$/RANKL-triggered differentiation under hPLEV-F. For quantification of resorption activity, BMC were seeded on a calcium phosphate lined and FACS coated plate and differentiated under M-CSF/RANKL- or -/TNF$\alpha$-conditions with or without hPLEV-F-treatment. Statistical analyses: Student's t-test; *p $\leq$ 0.05 (in A); #p $\leq$ 0.05 (compared to respective control).

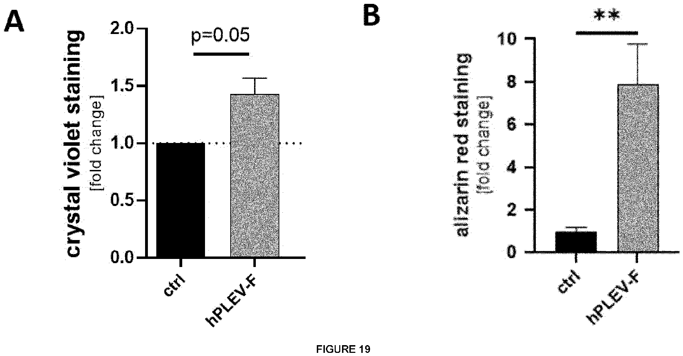

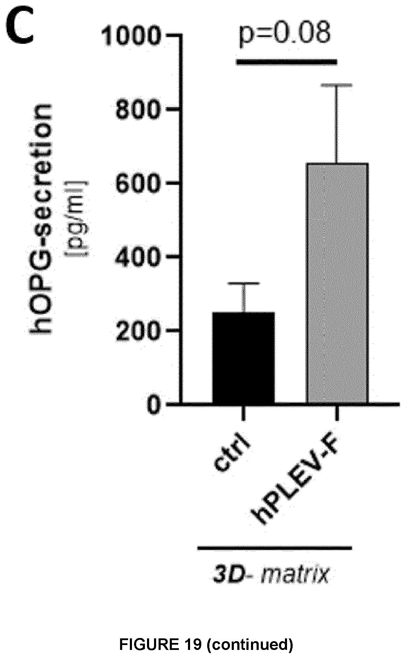

**Figure 19** - hPLEV-F-treatment enhances proliferation and differentiation and increases OPG-secretion from on the $\beta$-TCP-collagen composite of the disclosure -seeded with human osteoblasts. Primary human osteoblasts were cultured in hPLEV-F-supplemented proliferation medium and proliferation was quantified by crystal violet staining. After cell lysis, incorporated crystal violet was measured and depicted as fold change of control (A). Osteoblasts were differentiated for 14 days under hPLEV-treatment and quantified for mineralization in an alizarin red assay and calculated as fold change of control (B). Secretion of OPG as inhibitor of RANKL-mediated osteoclastogenesis was measured in an ELISA-assay after 14 days (C). Statistical analyses: Student's t-test; **p $\leq$ 0.01

Detailed Description of the Disclosure

The platelet-derived extracellular vesicle of the present disclosure, also called "EVs" (extracellular vesicle) is used as bone replacement material and/or as bone rebuilding material.

In another embodiment the platelet-derived extracellular vesicle as disclosed herein is used as a stimulator of osteoblast activity and/or inhibitor of osteoclast activity.

"Platelet-derived EVs" are small extracellular vesicles released by platelets, which are blood cells involved in clotting and wound healing. They are secreted by various cells, including platelets, and play important roles in intercellular communication and the regulation of physiological processes. Platelet-derived EVs comprise exosomes, macrovesicles, microvesicles und apoptotic bodies. The platelet-derived EVs according to the present disclosure may be purified to comprise only EVs of a certain diameter.

In one embodiment the platelet-derived extracellular vesicles (EVs) according to the present disclosure have a diameter of at least 50 nm, of at least 75 nm, of at least 90 nm, or of at least 100 nm. In one embodiment the platelet-derived EVsaccording to the present disclosure have a diameter of 250 nm or less, of 225 nm or less, of 215 nm or less, or of 200 nm or less. In one embodiment the platelet-derived EVs according to the present disclosure have a diameter from 50 to 250 nm, from 75 to 225 nm, from 90 to 215 nm, or from 100 to 200 nm.

Thus, in one embodiment, the platelet-derived EVs according to the present disclosure only comprise exosomes and microvesicles. In one embodiment, the proportion of exosomes within all vesicles present in the platelet-derived EVs according to the present disclosure, is at least 50%, at least 60%, at least 70%, at least 80%, at least 90%, at least 95%, or at least 98%. In one embodiment, platelet-derived EVs comprising only exosomes is used as bone replacement material and/or as bone rebuilding material.

The platelet-derived EVs according to the present disclosure contain various bioactive molecules, including proteins, lipids, nucleic acids (such as microRNAs), and growth factors. These molecules can influence the behavior and function of recipient cells by altering gene expression, signaling pathways, and cellular processes.

In the context of the present disclosure the platelet-derived EVs according to the present disclosure are of human origin.

Platelets, also named thrombocytes, are irregular, disc-shaped elements in the blood, that assist in blood-clotting. During normal blood-clotting, the platelets agglutinate by aggregation. Although platelets are often classified as blood cells, they have no nucleus. They derive from large cells called megakaryocytes located/sited in bone marrow.

For production of human platelet lysates (hPL), platelets are lysed and by this release their inner content into the surrounding plasma. Lysis of platelets can be achieved by freeze and thaw cycles. Suitable protocols can be found in the prior art. One biologic component that is contained to a high content in human platelet-lysates are extracellular vesicles. These are tiny, membrane-bound particles released from cells into the extracellular environment.

Human platelet lysates (hPL) may contain high amounts of EVs, secreted by living thrombocytes during storage time of platelet-concentrate-products. During a storage time of nearly one week at 20-24°C, cells continue secreting their specific EVs into the surrounding plasma. Thus, hPL contains platelet-derived EVs that were released from living thrombocytes during the period of storage of thrombocyte-concentrates (20-24°C) before reaching the expiry date after 4-6 days. Thus, the hPL contains a significant fraction of platelet-derived EVs. Thus, in one embodiment the present disclosure relates to EVs derived from thrombocyte-concentrates. Within this disclosure they are also termed "platelet-derived extracellular vesicles".

The platelet-derived EVs according to the present disclosure are particularly notable for their role in cell-to-cell communication. They can carry a wide range of molecules, such as proteins, lipids, RNA, and DNA, from their cell of origin to other cells. This cargo can alter the function or behavior of recipient cells, making the platelet-derived EVs according to the present disclosure significant in various physiological and pathological processes, including immune responses, cancer progression, and the spread of infectious agents.

The platelet-derived EVs according to the present disclosure function as a vehicle for various biomolecules including lipids, proteins (e.g. transcription factors, cytokines, growth factors) and nucleic acids such as mRNA, microRNA (miRNA) or even small amounts of DNA.

Thus, the platelet-derived EVs of the present disclosure contain nucleic acids including mRNA, miRNA and a large variety of other small noncoding RNA species, including RNA transcripts overlapping with protein coding regions, repeat sequences, structural RNAs, tRNA, rRNA, vault RNA, Y RNA, and small interfering RNAs (siRNA) as well as mitochondrial DNA, and short DNA sequences of retrotransposons.

Thus, in one embodiment the present disclosure relates to platelet-derived EVs comprising nucleic acids selected from the group consisting of mRNA, miRNA and other small noncoding RNA species, including RNA transcripts overlapping with protein coding regions, repeat sequences, structural RNAs, tRNA, rRNA, vault RNA, Y RNA, small interfering RNAs (siRNA), mitochondrial DNA, and short DNA sequences of retrotransposons, as well as combinations thereof.

Lipid components of exosomes include membrane lipids such as sphingomyelin, phosphatidylcholine, phosphatidylethanolamine, phosphatidylserine, gangliosides (GM3), phosphatidylinositol, prostaglandins and lysobisphosphatidic acid.

Thus, in one embodiment the present disclosure relates to platelet-derived EVs comprising membrane lipids such as sphingomyelin, phosphatidylcholine, phosphatidylethanolamine, phosphatidylserine, gangliosides (GM3), phosphatidylinositol, prostaglandins and lysobisphosphatidic acid, or a combination thereof.

Thus, in one embodiment the present disclosure relates to platelet-derived EVs comprising lipids, proteins (e.g. transcription factors, cytokines, growth factors) and nucleic acids such as mRNA, microRNA (miRNA) or even small amounts of DNA, or a combination thereof.

The platelet-derived EVs of the present disclosure may further comprise proteins and peptides.

The platelet-derived EVs of the present disclosure comprise membrane transporters and fusion-proteins (e.g., GTPases such as Rab5, annexins, and flotillins), heat shock proteins (e.g., HSC70), tetraspanins (e.g., CD9,CD63, and CD81), proteins from MVB-biogenesis (e.g., Alix and TSG101), lipid-related proteins and phospholipases, cytoskeleton proteins (actins, cofilin-1, ezrin/radixin/moesin, profilin-1, and tubulins), metabolic enzymes, and ribosomal proteins. Several proteins may be recognized as exosomal markers, among which the tetraspanins (e.g. CD63, CD81) and TSG101, a protein of the ESCRT-complex, are the most commonly used markers for detection.

Specific CD markers for the characterization of platelets are the surface markers CD9, CD41b, CD42a, CD42b and CD61 that appear on the platelet surface before activation. There are also markers that appear on the platelet surface during activation such as CD62P, CD63 and Syntenin. EVs, in particular exosomes, from platelets carry surface-markers corresponding to surface-markers on the secreting cell-entities, i.e. the platelets. The platelet-derived EVs of the present disclosure can therefore be identified by the expression of typical platelet surface markers.

The platelet-derived EVs of the present disclosure may be characterized by being positive for at least one biomarker selected from the group consisting of CD9, CD41b, CD42a, CD42b, CD61, CD62P, CD63, von-Willebrand-factor, Syntenin, TSG101, and a protein of the ESCRT-complex, or a combination thereof.

The platelet-derived EVs of the present disclosure may be characterized by being positive for at least one biomarker selected from the group consisting of CD41 (integrin alpha IIb), CD61 (integrin beta 3) and von-Willebrand-factor, or a combination thereof.

In one embodiment the platelet-derived extracellular vesicle according to the present disclosure is characterized by being positive for at least one biomarker selected from the group consisting of CD9, CD41b, CD42a, CD42b, CD61, CD62P, CD63, von-Willebrand-factor, Syntenin, TSG101, and a protein of the ESCRT-complex, as well as a combination thereof.

The platelet-derived EVs according to the present disclosure have been found to comprise one or more osteogenic proteins which can facilitate the growth and/or re-growth of bone tissue.

In one embodiment the platelet-derived extracellular vesicle according to the present disclosure is characterized by being positive for at least one growth factor selected from the group consisting of latent-transforming growth factor beta-binding protein, hepatocyte growth factor-like protein, and insulin-like growth factor-binding protein, as well as a combination thereof.

In one embodiment the platelet-derived extracellular vesicle according to the present disclosure comprises at least one osteogenic protein selected from the group consisting of OP-1, OP-2, OP-3, BMP2, BMP3, BMP4, BMP5, BMP6, BMP-7, BMP9, BMP10, BMP11, BMP12, BMP14, BMP15, BMP16, IGF, TGF, PDGF-BB, GDF1, GDF3, GDF5, GDF6, GDF7, GDF8, GDF9, GDF10, and GDF11, and a combination thereof.

Preferably, the source hPL for the platelet-derived EVs is produced according to GMP-conditions and preferably it has been manufactured in accordance with the German Drug Law (AMG). The hPL may originate from single or pooled donor-donated platelets. It might be preferred to use hPL originating from blood donors at certain age, e.g. from blood donors between 10-60 years, 18-50 years, 18-40 years, 18-30 years, or 18-20 years. In the context of precision medicine, it might be of advantage to treat a patient with human platelet-derived EVs of the present disclosure originating from its own blood donation. According to a preferred embodiment of the present disclosure the hPL originates from healthy individuals.

Thus, in one embodiment the platelet-derived extracellular vesicle for use as bone replacement and/or bone rebuilding material is of human origin. In one embodiment the platelet-derived extracellular vesicle for use as bone replacement and/or bone rebuilding material is from a subject's own blood donation. In one embodiment the platelet-derived extracellular vesicle for use as bone replacement and/or bone rebuilding material is from healthy human individuals.

hPL or a fraction that is enriched for hPL can be obtained from platelets that have been incubated before the lysis with one or more compounds known as thrombocyte activators from the prior art, e.g. prior art related to PRP therapy, that activate the platelets and thus improve the quality of the inventive preparation. According to one embodiment of the hPL might be derive from a Human Umbilical Cord Blood. Human Umbilical Cord Blood Platelet Lyse preparations are known from the prior art (e.g. US 8,501,170 B2, Parazzi, V., C. Lavazza, et al. (2015) or Forte, D., M. Ciciarello, et al. (2015).

The hPLEV-F were resolved in 0.9% NaCl-solution in a concentration from 1 mg/ml to 5 mg/ml, from 2 mg/ml to 4 mg/ml, or about 3,75 mg/ml. It has been found that at effective concentration of from 150 to 300, from 175 to 250, or from

about 187,5 $\mu$g hPLEV-F/ml medium, several effects can be seen:

- The inflammatory stimulus is reduced with addition of EVs. This results in a reduction of bone resorption.

- In wound healing scratch assay an increased healing could be seen. This shows that EVs trigger and improve wound healing.

- The osteoblast differentiation and mineralization is increased with EVs. This shows that EVs increase bone metabolism and improve bone formation and/or bone rebuilding.

- EVs are not toxic.

These features render platelet-derived EVs very useful for use in bone replacement and/or bone rebuilding.

In one embodiment the platelet-derived EVs of the present description reduces bone reduction and/or may induce bone growth by stimulating the Wnt-signaling pathway, stimulating the Bone Morphogenetic Protein (BMP) signaling pathway, influencing the Notch Signaling Pathway by changing the balance rather to bone formation than bone resorption, influencing the MAPK (Mitogen-Activated Protein Kinase) signaling pathway by changing the balance rather to bone formation than bone resorption, influencing the TGF-beta (Transforming Growth Factor-beta) Signaling Pathway by changing the balance rather to bone formation than bone resorption, influencing the Hedgehog Signaling Pathway by changing the balance rather to bone formation than bone resorption, or reducing the RANK/RANKL/OPG signaling pathway.

In one embodiment the platelet-derived EVs of the present description reduce bone reduction and/or induce bone growth by stimulating at least one of the signaling pathways selected from the group consisting of MAPK14/p38$\alpha$, MAPK3/ERK1, MAPK1/ERK2, AKT1, and CDK5; as well as combinations thereof.

The pathways involving MAPK14/p38$\alpha$, MAPK3/ERK1, MAPK1/ERK2, AKT1, and CDK5 are crucial regulators of a wide range of cellular processes, from stress response and growth to survival and differentiation. Each of these proteins functions as part of larger signaling cascades, which help cells respond to both external and internal signals in order to maintain homeostasis, promote growth, or initiate defense mechanisms.

MAPK14, also known as p38$\alpha$, is a key component of the p38 MAPK pathway, which is primarily activated by stress signals such as UV radiation, heat, or pro-inflammatory cytokines. Once activated, this pathway plays a vital role in managing the cell's response to stress by regulating the production of pro-inflammatory molecules like TNF-$\alpha$ and IL-1, controlling apoptosis, and influencing cell cycle dynamics. In the context of disease, p38$\alpha$ can exhibit both pro- and anti-tumorigenic properties, depending on the cellular environment, making it an interesting target in cancer research as well as inflammatory diseases. In one embodiment the platelet-derived EVs of the present description therefore positively influence cell's response to stress by regulating the production of pro-inflammatory molecules.

On the other hand, MAPK3/ERK1 and MAPK1/ERK2 are central to the ERK/MAPK signaling pathway, which is highly responsive to growth factors and mitogens. ERK1 and ERK2 share overlapping roles in promoting cell proliferation, survival, and differentiation. Upon activation by upstream kinases like MEK1/2, ERK proteins translocate to the nucleus, where they influence gene expression to drive cell division and prevent apoptosis. This pathway is essential for normal cellular growth and development but is often hijacked in cancerous cells. Mutations in upstream components such as RAS and BRAF can lead to the overactivation of the ERK pathway, driving uncontrolled cell proliferation in various cancers. Notably, ERK2 has been shown to play specific roles in neuronal differentiation and synaptic plasticity, underscoring its importance in brain development and function. In one embodiment the platelet-derived EVs of the present description therefore positively influence cell's gene expression to drive cell division and prevent apoptosis.

AKT1, part of the PI3K/AKT signaling pathway, is another major player in cell survival and metabolism. When stimulated by growth factors, AKT1 is activated via the PI3K pathway and plays a central role in preventing apoptosis, regulating glucose uptake, and promoting protein synthesis and cell growth. AKT1 is particularly important in maintaining cell survival by phosphorylating and inactivating pro-apoptotic proteins such as BAD. Its role extends to the regulation of glucose metabolism and angiogenesis through targets like GSK3$\beta$ and VEGF, making AKT1 a crucial player not only in normal cell physiology but also in cancer. Aberrations in the PI3K/AKT pathway, including loss of the tumor suppressor PTEN or gain-of-function mutations in PI3K, are common in many cancers, leading to enhanced cell survival and tumor progression. In one embodiment the platelet-derived EVs of the present description therefore positively influence maintaining cell survival by phosphorylating and inactivating pro-apoptotic proteins.

Finally, CDK5 is not involved in cell cycle regulation like other cyclin-dependent kinases (CDKs). Instead, it is crucial for neuronal development and function. Activated by its co-factors p35 or p39, CDK5 regulates processes like synaptic activity, axon guidance, and neuronal survival. Dysregulation of CDK5 activity, particularly through the cleavage of p35 to p25, is implicated in neurodegenerative diseases such as Alzheimer's, where it contributes to the hyperphosphorylation of tau, a hallmark of the disease. While CDK5's primary role is in the nervous system, emerging research suggests that it may also have a role in cancer metastasis and survival, further expanding its relevance beyond neurobiology. In one embodiment the platelet-derived EVs of the present description therefore positively influence cell survival.

In summary, in one embodiment the platelet-derived EVs of the present description therefore positively influence these signalling molecules which are part of interconnected networks that regulate essential cellular functions. MAPK14/-p38$\alpha$ governs stress responses, inflammation, and apoptosis, while MAPK3/ERK1 and MAPK1/ERK2 drive cell proliferation and survival in response to growth signals. AKT1 promotes cell growth and prevents apoptosis, playing a pivotal role in both metabolism and cancer biology, and CDK5 is crucial for neuronal function and has links to both neurodegenerative diseases and cancer.

The present disclosure also pertains to a composite material for use as bone replacement and/or bone rebuilding material, characterized in comprising the platelet-derived EVs according to present disclosure, at least one inorganic component and at least one organic component. The composite material without the EVs is also called "scaffold material" hereinunder.

In one embodiment the composite material according to the present disclosure comprises an inorganic component, wherein the inorganic component, is selected from the group consisting of calcium phosphate; sodium-, potassium-, calcium- and/or silicate-containing acid; neutral and/or alkaline bioglass; and glass ce-ramic, and a combination thereof; optionally in form of a granulate, the granulate made from a material selected from the group consisting of calcium phosphate, sodium-, po-tassium-, calcium- and/or silicate-containing acid, neutral and/or alkaline bioglass, and glass ceramic, and a combination thereof; wherein the granulate optionally comprising biologically active, polygon-broken, rounded particles.

In one embodiment the composite material according to the present disclosure comprises calcium phosphate which is selected from the group consisting of $\beta$-tricalcium phosphate, preferably $\beta$-tricalcium phosphate from 0 to 15% by weight, from 0.1 to 14% by weight, from 0.25 to 12 by weight, from 0.5 to 10% by weight, or from 0.75 to 8% by weight of sodium magnesium silicate glass, monocalcium phosphate monohydrate, anhydrous monocalcium phosphate, dicalcium phosphate dihydrate, anhydrous dicalcium phosphate, tetra-calcium phosphate, whitlockite, octacalcium phosphate, hydroxyapatite, oxyapatite, type A carbonatapatite, type B carbonatapatite, calcium-deficient hydroxyapatite, amorphous calcium phosphate, amorphous carbonate-containing calcium phosphate, and calcium alkali orthophosphate glass ceramic, and a combination thereof.

In one embodiment the composite material according to the present disclosure comprises an organic component which is selected from the group consisting of a polymer, such as polypeptide, polysaccharide, and poly-lipid, and a combination thereof.

In one embodiment the composite material according to the present disclosure comprises an organic component which is selected from the group consisting of gelatine, collagen, glycosaminoglycan, hyaluronan, sodium hyaluronate, calcium hyaluronate, cellulose such as methyl-cellulose, ethylcellulose, propylcellulose, hydroxypropylcellulose, hydroxyethylcellulose, starch, dextran, hydroxyethyl starch, alginate, polyethylene glycol, albumin, and chitosan, or a combination thereof.

In one embodiment the composite material according to the present disclosure comprises at least a hydrogel and a platelet-derived EV . Such a composite material is used for injection into a patient.

In one embodiment the composite material according to the present disclosure comprises an organic component which is selected from the group consisting of organically processed or demineralized allogeneic or xenogeneic bone material, synthetic polypeptide, parathyroid hormone, and osteogenic protein, or a combination thereof.

In one embodiment the composite material according to the present disclosure the organic component comprises an osteogenic protein which is selected from the group consisting of OP-1, OP-2, OP-3, BMP2, BMP3, BMP4, BMP5, BMP6, BMP-7, BMP9, BMP10, BMP11, BMP12, BMP14, BMP15, BMP16, IGF, TGF, PDGF-BB, GDF1, GDF3, GDF5, GDF6, GDF7, GDF8, GDF9, GDF10, and GDF11, and a combination thereof.

In one embodiment the composite material according to the present disclosure is characterized by an inorganic component having at least one of the features selected from the group consisting of:

- being a ceramic component comprising calcium, phosphate, $SiO_2$, $MgO$ and $Na_2O$,

- being a granulate whose particles have a $d_{50}$ value of at least 10 $\mu$m,

- being present in the composite material from 15 to 80 wt.-%, from 20 to 70 wt.-%, or from 30 to 60 wt.-%, and

- having an interconnecting, open multi-porosity with micro-, meso- and macropores (pore diameter from 0.05 $\mu$m to 500 $\mu$m, from 0.1 $\mu$m to 300 $\mu$m, from 0.1 $\mu$m to 50 $\mu$m, or from 0.1 $\mu$m to 30 $\mu$m) and a total porosity of from at least 25%, at least 35%, at least 45%, at least 55%, at least 65%, or at least 75%, from at least 25% to up to 75%, from at least 35% to up to 65%, as well as combinations thereof.

The average pore size as well as the porosity may be measured by gas adsorption (e.g., Brunauer-Emmett-Teller or BET Method) according to ISO 9277:2010. In one embodiment the BET surface area is from 50 to 5,000 $m^2/g$, from 100 to 2,000 $m^2/g$, from 250 to 1,500 $m^2/g$, from 500 to 1,200 $m^2/g$, or from 750 to 1,000 $m^2/g$.

Interconnected porosity refers to the volume fraction of pores within a material that are connected to each other, forming a continuous network. This allows fluids (gases or liquids) to pass through the material. In other words, interconnected porosity represents the portion of the pore structure that contributes to permeability. Open porosity refers to the volume fraction of pores that are accessible from the surface of the material, meaning they are exposed to the external environment. Open porosity includes all pores that can interact with the surroundings, whether or not they are connected to a network that extends through the entire material.

X-ray Microtomography (Micro-CT) may be used to measure the spatial distribution and connectivity of pores and to differentiate between interconnected and open pores.

In one embodiment the composite material according to the present disclosure comprises an inorganic component having one or more or all of the following features

a) being present in the composite material with about 80 wt.% - 90 wt.-%, such as about 85 wt.-%, having a particle size from 1000-2000 $\mu$m, a total porosity of about 60% to 90%, such as at least 65%, and a pore-size of from 0.1 to 500 $\mu$m, from 0.5 to 500 $\mu$m, from 1 to 500 $\mu$m, or from 5 to 500 $\mu$m;

b) being present in the composite material with about 75 wt.% - 85 wt.-%, such as about 80 wt.-%, having a particle size from 1000-2000 $\mu$m, a total porosity of about 60% to 90%, such as at least 65%, and a pore-size of from 0.1 to 500 $\mu$m, from 0.5 to 500 $\mu$m, from 1 to 500 $\mu$m, or from 5 to 500 $\mu$m;

c) being present in the composite material with about 10 wt.% - 20 wt.-%, such as about 15 wt.-%, having a particle size from 150-500 $\mu$m, a total porosity of about 60% to 90%, such as at least 65%, and a pore-size of from 0.1 to 300 $\mu$m from 0.5 to 300 $\mu$m, from 1 to 300 $\mu$m, or from 5 to 300 $\mu$m; and

d) being present in the composite material with about 1 wt.% - 10 wt.-%, such as about 5 wt.-%, having a particle size from 150-500 $\mu$m, a total porosity about 30% to 50%, such as of at least 35%, and a pore-size of from 0.1 to 30 $\mu$m, from 0.5 to 30 $\mu$m, from 1 to 30 $\mu$m, or from 2 to 30 $\mu$m.

The scaffold material of the composite according to the present disclosure consists of components of natural bone, such as collagen and beta-tri-calcium phosphate (beta-TCP). These serve as basic building blocks for bone formation. The EVs in the composite according to the present disclosure accelerate the differentiation of osteoblasts and lead to faster mineralization. Surprisingly the beneficial effects for bone growth and bone protection are higher for the composite comprising the EVs than for the composite without EVs or cell samples with EVs alone. Thus, the effects are synergistically enhanced.

For example, the composites of the present disclosure show good EV-binding. The colonization of the composite material by cells is improved. Cell proliferation and migration is increased, as well as mineralisation. Osteocalcin is increasingly active in the material. At the same time inflammation and RANKL-secretion is reduced. OPG-secretion and bone protection are increased. The basic building blocks for bone formation are faster metabolized. Last, but not least, the inflammatory process are reduced.

In one embodiment the EVs and/or the composite of the present disclosure is used for the treatment of a patient in order to increase and/or decrease one or more of the biomarkers within said patient. In particular, the EVs and/or the composite of the present disclosure is used for the treatment of a patient in need of bone replacement and/or bone rebuilding material.

In one embodiment the EVs and/or the composite of the present disclosure is used for the treatment of a patient in need of increasing at least one biomarker selected from Homeobox Protein Hox-B1 (Hmbox1), Prostaglandin-Endoperoxide Synthase 2 (Ptgs2), Integrin Alpha-2 (Itga2), and Progressive Ankylosis Protein Homolog (Ankh). In one embodiment the EVs and/or the composite of the present disclosure is used for the treatment of a patient in need of increasing at least one of the biomarkers selected from Homeobox Protein Hox-B1 (Hmbox1), Prostaglandin-Endoperoxide Synthase 2 (Ptgs2), Integrin Alpha-2 (Itga2), and Progressive Ankylosis Protein Homolog (Ankh), as measured as $Log_2$-fold change, as compared to osteoblasts cells by at least 0.4-fold, at least 0.5-fold, at least 0.6-fold, at least 0.7-fold, at least 0.8-fold, at least 0.9-fold, or at least 1-fold.

In one embodiment the EVs and/or the composite of the present disclosure is used for the treatment of a patient in need of increasing osteoblast cell migration in the composite material as compared to control osteoblast cells by up to 3-fold, up to 2.7-fold, up to 2.3-fold, or up to 2.0-fold. In one embodiment the EVs and/or the composite of the present disclosure is used for the treatment of a patient in need of increasing osteoblast cell migration in the composite material of the present disclosure as compared to control osteoblast cells without the composite material of the present disclosure by between 0.4-fold and 3-fold, between 0.5-fold and 2.7-fold, between 0.6-fold and 2.3-fold, between 0.6-fold and 2.1-fold, or between 0.7-fold and 2.0-fold.

"Log$_2$-fold change" is defined herein to quantify changes in gene expression or protein levels. It represents the ratio between two values, such as gene expression in an experimental group versus a control group. If the fold change is 2, it means the gene is expressed twice as much in the experimental group compared to the control group. A log$_2$ value of 1 means a twofold increase (fold change of 2), and a log$_2$ value of -1 means a twofold decrease (fold change of 0.5). Further examples:

- A fold change of 2 (doubling) corresponds to a log$_2$ fold change of 1.

- A fold change of 0.5 (halving) corresponds to a log$_2$ fold change of -1.

- A fold change of 1 (no change) corresponds to a log$_2$ fold change of 0.

In summary, "Log 2 fold change" indicates how much the value of a gene or protein has changed compared to a reference (e.g., control), using the logarithm base 2.

In one embodiment the EVs and/or the composite of the present disclosure is used for the treatment of a patient in need of decreasing at least one biomarker selected from Collagen Type XII Alpha 1 Chain (Col12A1), Cell Migration-Inducing Hyaluronidase 1 (CEMIP), S100 Calcium-Binding Protein A6 (S100a6), and Growth Arrest-Specific 6 (Gas6). In one embodiment the EVs and/or the composite of the present disclosure is used for the treatment of a patient in need of decreasing at least one biomarker selected from Collagen Type XII Alpha 1 Chain (Col12A1), Cell Migration-Inducing Hyaluronidase 1 (CEMIP), S100 Calcium-Binding Protein A6 (S100a6), and Growth Arrest-Specific 6 (Gas6),as measured as Log$_2$-fold change, as compared to osteoblast cells by at least 0.4-fold, at least 0.5-fold, at least 0.6-fold, at least 0.7-fold, at least 0.8-fold, at least 0.9-fold, or at least 1-fold. ). In one embodiment the EVs and/or the composite of the present disclosure is used for the treatment of a patient in need of decreasing at least one biomarker selected from Collagen Type XII Alpha 1 Chain (Col12A1), Cell Migration-Inducing Hyaluronidase 1 (CEMIP), S100 Calcium-Binding Protein A6 (S100a6), and Growth Arrest-Specific 6 (Gas6), as measured as Log$_2$-fold change, by up to 3-fold, up to 2.7-fold, up to 2.3-fold, or up to 2.0-fold. ). In one embodiment the EVs and/or the composite of the present disclosure is used for the treatment of a patient in need of decreasing at least one biomarker selected from Collagen Type XII Alpha 1 Chain (Col12A1), Cell Migration-Inducing Hyaluronidase 1 (CEMIP), S100 Calcium-Binding Protein A6 (S100a6), and Growth Arrest-Specific 6 (Gas6), as measured as Log$_2$-fold change, of between 0.4-fold and 3-fold, between 0.5-fold and 2.7-fold, between 0.6-fold and 2.3-fold, between 0.6-fold and 2.1-fold, or between 0.7-fold and 2.0-fold.

In one embodiment the EVs and/or the composite of the present disclosure is used for the treatment of a patient in need of increasing a biomarker, when being stimulated with TNFα. Wherein the at least one biomarker is selected from Prostaglandin-Endoperoxide Synthase 2 (Ptgs2), Progressive Ankylosis Protein Homolog (Ankh), Parvin Beta (Parvb), and Inhibin Subunit Beta A (Inhba). In one embodiment the EVs and/or the composite of the present disclosure is used for the treatment of a patient in need of increasing a biomarker selected from Prostaglandin-Endoperoxide Synthase 2 (Ptgs2), Progressive Ankylosis Protein Homolog (Ankh), Parvin Beta (Parvb), and Inhibin Subunit Beta A (Inhba), when being stimulated with TNFα, as measured as Log$_2$-fold change, as compared to osteoblast cells by at least 0.4-fold, at least 0.5-fold, at least 0.6-fold, at least 0.7-fold, at least 0.8-fold, at least 0.9-fold, or at least 1-fold. In one embodiment the EVs and/or the composite of the present disclosure is used for the treatment of a patient in need of increasing a biomarker selected from Prostaglandin-Endoperoxide Synthase 2 (Ptgs2), Progressive Ankylosis Protein Homolog (Ankh), Parvin Beta (Parvb), and Inhibin Subunit Beta A (Inhba), when being stimulated with TNFα up to 3-fold, up to 2.7-fold, up to 2.3-fold, or up to 2.0-fold. In one embodiment the EVs and/or the composite of the present disclosure is used for the treatment of a patient in need of increasing a biomarker selected from Prostaglandin-Endoperoxide Synthase 2 (Ptgs2), Progressive Ankylosis Protein Homolog (Ankh), Parvin Beta (Parvb), and Inhibin Subunit Beta A (Inhba), when being stimulated with TNFα. ofbetween 0.4-fold and 3-fold, between 0.5-fold and 2.7-fold, between 0.6-fold and 2.3-fold, between 0.6-fold and 2.1-fold, or between 0.7-fold and 2.0-fold.

In one embodiment the EVs and/or the composite of the present disclosure is used for the treatment of a patient in need of decreasing a biomarker, when being stimulated with TNFα. Wherein the at least one biomarker is selected from Collagen Type I Alpha 1 Chain (Col1a1), Collagen Type I Alpha 2 Chain (Col1a2), and Milk Fat Globule-EGF Factor 8 Protein (Mfge8). In one embodiment the EVs and/or the composite of the present disclosure is used for the treatment of a patient in need of decreasing a biomarker selected from Collagen Type I Alpha 1 Chain (Col1a1), Collagen Type I Alpha 2 Chain (Col1a2), and Milk Fat Globule-EGF Factor 8 Protein (Mfge8), when being stimulated with TNFα, as measured as Log$_2$-fold change, as compared to osteoblast cells by at least 0.4-fold, at least 0.5-fold, at least 0.6-fold, at least 0.7-fold, at least 0.8-fold, at least 0.9-fold, or at least 1-fold. In one embodiment the EVs and/or the composite of the present disclosure is used for the treatment of a patient in need of decreasing a biomarker selected from Collagen Type I Alpha 1 Chain (Col1a1), Collagen Type I Alpha 2 Chain (Col1a2), and Milk Fat Globule-EGF Factor 8 Protein (Mfge8), when being

stimulated with TNF$\alpha$, as measured as Log$_2$-fold change, up to 3-fold, up to 2.7-fold, up to 2.3-fold, or up to 2.0-fold. In one embodiment the EVs and/or the composite of the present disclosure is used for the treatment of a patient in need of decreasing a biomarker selected from Collagen Type I Alpha 1 Chain (Col1a1), Collagen Type I Alpha 2 Chain (Col1a2), and Milk Fat Globule-EGF Factor 8 Protein (Mfge8), when being stimulated with TNF$\alpha$, as measured as Log$_2$-fold change, of between 0.4-fold and 3-fold, between 0.5-fold and 2.7-fold, between 0.6-fold and 2.3-fold, between 0.6-fold and 2.1-fold, or between 0.7-fold and 2.0-fold.

In further embodiments the EVs and/or the composite of the present disclosure is used for the treatment of a patient in need of increasing and/or decreasing a biomarker selected form at least one of the biomarkers summarized in table 1 as well as combinations thereof.

In yet another embodiment the EVs and/or the composite of the present disclosure is used for the treatment of a patient in need of increasing and/or decreasing a biomarker in at least a specific x-fold manner, selected form at least one of the biomarkers summarized in table 1 as well as combinations thereof.

**Table 1 - Summary of biomarker characteristics.**

| Marker | State | Secretion | Content | Expression | x-fold change* | Comment | Figure |
|--------|-------|-----------|---------|------------|----------------|---------|--------|
| OPG | 4 days | | increased | | At least 5-fold | 2D vs. 2D EV | 8 |
| | 13 days | | increased | | At least 2.5-fold | 2D vs. 2D EV | 8 |
| | 4 days | | increased | | At least 3-fold | 3D vs. 3D EV | 8 |
| | 14 days | | increased | | At least 6-fold | 3D vs. 3D EV | 8 |
| | | | | decreased | 0.4-fold or less | 2D vs. 2D EV | 14 |
| | | increased | | | At least 3.7-fold | 3D vs. 3D EV | 15 |
| | | increased | | | At least 2-fold | 3D vs. 2D EV | 15 |
| TNF-$\alpha$ | | increased | | | At least 3-fold | 2D vs. 2D EV | 13 |
| | | | decreased | | 0.3-fold or less | 2D+ TNF$\alpha$ vs. 2D EV + TNF$\alpha$ | 14 |
| | 4 days | increased | | | At least 5-fold | 2D vs. 2D EV | 17 |
| | 13 days | increased | | | At least 3-fold | 2D vs. 2D EV | 17 |
| | 4 days | decreased | | | 0.9-fold or less | 3D vs. 3D EV | 17 |
| | 14 days | increased | | | At least 1.1-fold | 3D vs. 3D EV | 17 |
| RANKL | | decreased | | | 0.2-fold or less | 2D vs. 2D EV | 14 |
| | | decreased | | | 0.3-fold or less | 2D+TNF$\alpha$ vs. 2D EV+ TNF$\alpha$ | 14 |
| IL-10 | | increased | | | At least 2.1-fold | 2D vs. 2D EV | 13 |
| | | decreased | | | 0.3-fold or less | 2D vs. 2D EV | 14 |
| | | decreased | | | 0.6-fold or less | 2D+TNF$\alpha$ vs. 2D EV + TNF$\alpha$ | 14 |
| | 4 days | decreased | | | 0.9-fold or less | 2D vs. 2D EV | 17 |
| | 13 days | increased | | | At least 2-fold | 2D vs. 2D EV | 17 |
| | 4 days | decreased | | | 0.9-fold or less | 3D vs. 3D EV | 17 |
| | 14 days | decreased | | | 0.9 fold or less | 3D vs. 3D EV | 17 |

(continued)

| Marker | State | Secretion | Content | Expression | x-fold change* | Comment | Figure |
|---|---|---|---|---|---|---|---|
| IL1β |  | decreased |  |  | 0.2-fold or less | 2D vs. 2D EV | 14 |
|  |  | decreased |  |  | 0.4-fold or less | 2D+TNFα vs. 2D EV + TNFα | 14 |
|  | 4 days | decreased |  |  |  | 2D vs. 2D EV | 17 |
|  | 13 days | decreased |  |  | 0.9-fold or less | 2D vs. 2D EV | 17 |
|  | 4 days | decreased |  |  |  | 3D vs. 3D EV | 17 |
|  | 14 days | decreased |  |  | 0.3-fold or less | 3D vs. 3D EV | 17 |
| IL33 |  | decreased |  |  |  | 2D vs. 2D EV | 14 |
|  |  | decreased |  |  | 0.3-fold or less | 2D+TNFα vs. 2D EV + TNFα | 14 |
|  | 4 days | decreased |  |  |  | 2D vs. 2D EV | 17 |
|  | 13 days | decreased |  |  | 0.4-fold or less | 2D vs. 2D EV | 17 |
|  | 4 days | decreased |  |  | 0.1-fold or less | 3D vs. 3D EV | 17 |
|  | 14 days | decreased |  |  | 0.1-fold or less | 3D vs. 3D EV | 17 |
| IL6 |  | increased |  |  | At least 5-fold | 2D vs. 2D EV | 13 |
|  |  | increased |  |  | At least 3.5-fold | 2D vs. 2D EV | 14 |
|  |  | increased |  |  | At least 1.5-fold | 2D + TNFα vs. 2D EV + TNFα | 14 |
|  | 4 days | increased |  |  |  | 2D vs. 2D EV | 16 |
|  | 13 days | increased |  |  | At least 4-fold | 2D vs. 2D EV | 16 |
|  | 4 days | increased |  |  |  | 3D vs. 3D EV | 16 |
|  | 14 days | increased |  |  | At least1.5-fold | 3D vs. 3D EV | 16 |
| MIP2α |  | increased |  |  | At least 3-fold | 2D vs. 2D EV | 13 |
|  |  | increased |  |  | At least 2-fold | 2D vs. 2D EV | 14 |
|  |  | decreased |  |  | 0.9-fold or less | 2D+TNFα vs. 2D EV + TNFα | 14 |
|  | 4 days | increased |  |  |  | 2D vs. 2D EV | 16 |
|  | 13 days | increased |  |  | At least 2.6-fold | 2D vs. 2D EV | 16 |
|  | 4 days | decreased |  |  |  | 3D vs. 3D EV | 16 |
|  | 14 days | increased |  |  | At least 1.8-fold | 3D vs. 3D EV | 16 |
| M-CSF |  | decreased |  |  | 0.8 fold or less | 2D vs. 2D EV | 13 |
|  |  | decreased |  |  | 0.4-fold or less | 2D vs. 2D EV | 14 |
|  |  | decreased |  |  | 0.6-fold or less | 2D+TNFα vs. 2D EV + TNFα | 14 |
| Col1A1 |  |  |  | decreased | 0.8-fold or less | 2D vs. 2D EV | 14 |
| Runx2 |  |  |  | decreased | 0.4-fold or less | 2D vs. 2D EV | 14 |
| Ocn |  |  |  | decreased | 0.3-fold or less | 2D vs. 2D EV | 14 |

(* "x-fold change" was calculated in table 1 by the formula: amount of marker in control * x-fold-change = amount of marker in sample; for example, a "3-fold change" means that the amount of the respective marker was 3-fold increased as compared to control; "0.7-fold change" means that that the amount of the respective marker was 0.7-fold decreased as compared to control.)

Thus, in one embodiment the EVs and/or the composite of the present disclosure is used for the treatment of a patient in need of increasing theosteoblast cell as compared to osteoblast cells ("x-fold change" as in table 1) by at least 1.1-fold, at least 1.2-fold, at least 1.5-fold, at least 2-fold, at least 2.2-fold, at least 2.5-fold, or at least 3-fold. In another embodiment the EVs and/or the composite of the present disclosure is used for the treatment of a patient in need of increasing the osteoblast cell migration as compared to control osteoblast cells by up to 7-fold, up to 6-fold, up to 5-fold, or up to 4-fold. In another embodiment the EVs and/or the composite of the present disclosure is used for the treatment of a patient in need of increasing the cell migration in as compared to control osteoblast cells by between 1.1-fold and 7-fold, between 1.2-fold and 6-fold, between 1.5-fold and 5-fold, between 1.8-fold and 4-fold, or between 2-fold and 3-fold.

In one embodiment the EVs and/or the composite of the present disclosure is used for the treatment of a patient in need of decreasing osteoclast activity as measured by TRAP as compared to control osteoclast cells ("x-fold change" as in table 1) by at least 1.1-fold, at least 1.2-fold, at least 1.5-fold, at least 2-fold, at least 2.2-fold, at least 2.5-fold, or at least 3-fold. In one embodiment the EVs and/or the composite of the present disclosure is used for the treatment of a patient in need of decreasing osteoclast activity as measured by TRAP as compared to control osteoclast cells ("x-fold change" as in table 1) by up to 7-fold, up to 6-fold, up to 5-fold, or up to 4-fold. In one embodiment the EVs and/or the composite of the present disclosure is used for the treatment of a patient in need of decreasing osteoclast activity as measured by TRAP as compared to control osteoclast cells ("x-fold change" as in table 1) by between 1.1-fold and 7-fold, between 1.2-fold and 6-fold, between 1.5-fold and 5-fold, between 1.8-fold and 4-fold, or between 2-fold and 3-fold.

Other effects present in the composites of the present disclosure which may be used in a method of treating a patient in need thereof:

- Human Osteoblasts show increased proliferation, increased differentiation and improved interaction with the composite material.

- Human fibroblasts show improved activity, leading to an up to 4-fold increased wound healing, such as for example of 6h vs. 22h without EVs.

- Human Osteoclasts show reduced activity, thereby the bone degradation is reduced.

In one embodiment the present disclosure pertains to a method for production of a composite comprising the steps of:

a. Providing the composite according to the disclosure;

b. Adding at least one platelet-derived EVs suspended in a buffer, such as phosphate buffered saline (PBS, pH 7.4) to a final EV concentration from $1 \times 10^8$ to $1 \times 10^9$ particles/mL, such as $3.75 \times 10^8$ particles/mL,

In one embodiment the present disclosure pertains to a method for production of a composite comprising the steps of:

a. adding at least one organic component to water to form a suspension;

b. adding at least one inorganic component; and, optionally, a crosslinking agent; to the suspension of step a.) and mix the components;

c. transferring the solution from step b.) into a mold and then drying the mixture by lyophilization to form the composite material;

d. adding at least one platelet-derived EVs to water or buffer (pH 7.4);

e. immersing the composite material of step c.) with the solution of step d.).

In another embodiment the present disclosure pertains to method for production of a composite comprising the steps of:

a. adding at least one organic component to a water-containing solution (e.g. a buffer) to form a suspension;

b. adding at least one inorganic component; and, optionally, a crosslinking agent; to the suspension of step a.) and mixing the components;

c. transferring the solution from step b.) into a mold and then drying the mixture by lyophilization to form the composite material;

d. adding at least one platelet-derived EVs to a solution comprising a salt, such as sodium chloride;

e. Immersing the composite material of step c.) into the solution of step d.).

In yet another embodiment the present disclosure pertains to method for production of a composite comprising the steps of:

a. adding at least one organic component to a water-containing solution to form a suspension;

b. adding at least one inorganic component; and, optionally, a crosslinking agent; to the suspension of step a.), further adding at least one platelet-derived EVs to a solution comprising a salt, such as sodium chloride, and mixing the components;

c. transferring the solution from step b.) into a mold and then drying the mixture by lyophilization to form the composite material;

In yet another embodiment the present disclosure pertains to method for production the composite according to the disclosure comprising the steps of:

a. Preparing a synthetic polymer, such as a non-toxic and biocompatible polymer, such as polyvinylpyrrolidone (PVP; Mw 360 kDa) in solution in an adequate solvent, such as in trifluoroethane at 10% w/v,

b. Adding at least one platelet-derived EVs suspended in a buffer, such as phosphate buffered saline (PBS) to a final EV concentration from $1 \times 10^8$ to $1 \times 10^9$ particles/mL, such as about $3.75 \times 10^8$ particles/mL,

c. Electrospinning at a slow flow rate from 0.1 mL/h to 10 mL/h, such as about 1 mL/h, at a spinneret-to-collector distance from 5 cm to 30 cm, such as about 18 cm and an applied voltage from 1kV to 15 kV, such as about 8 kV.

d. Thereby obtaining fibers of the composite which then can be used for further processing.

In yet another embodiment the present disclosure pertains to method for production the composite according to the disclosure by manufacturing printing pastes and inks for three-dimensional printing of bone replacement material including extracellular vesicles.

In one embodiment the extracellular vesicles of the disclosure are incorporated in a printing paste or printing ink, in combination with bone replacement material composed of collagen/beta-TCP to create a three-dimensional printed product for bone regeneration or bone replacement or bone rebuilding. Alternatively, the extracellular vesicles are added to the printed bone material e.g. by coating/soaking. After three-dimensional printing such composite material can be further processed by lyophilization/freeze drying to increase extracellular vesicle stability.

Such a method comprises the steps of

a). Preparing a collagen paste, comprising the steps of dissolving collagen in an acidic solution, mixing the dissolved collagen with the inorganic material according to the present disclosure selected from the group consisting of calcium phosphate, sodium-, potassium-, calcium- and/or silicate-containing acid, neutral and/or alkaline bioglass, and glass ceramic, and a combination thereof; wherein the granulate comprises biologically active, polygon-broken, rounded particles and, optionally, adding the EVs of the present disclosure suspended in buffer solution.

b.) Optionally, storing the paste at 4°C until use;

c.) Using the paste in a 3D screen printing unit in order to three-dimensional print the bone replacement material.

Alternatively, the extracellular vesicles are placed onto the printed bone replacement material after printing.

The step of applying the EVs can be performed by any suitable technology, including spraying, soaking, coating, imbibing, impregnating, applying and subsequently freeze-drying, nanosol technology, applying and subsequently exposing the printed bone replacement material to negative pressure or vacuum, etc..

In some embodiments a crosslinking agent is selected from the group consisting of an organic acid, or an aqueous or alcoholic solution of an organic acid, such as formic acid, acetic acid, propionic acid, an aqueous or alcoholic solution of methanoic acid (formic acid), ethanoic acid (acetic acid), propanoic acid (propionic acid), butanoic acid (butyric acid), n-butyric acid, isobutyric acid, pentanoic acid (valeric acid), n-valeric acid, iso-valeric acid, 2-methylbutyric acid, pivalic acid, oxalic acid, malonic acid, succinic acid, glutaric acid, adipic acid, pimelic acid, hydroxyacetic acid (glycolic acid), hydroxypropionic acid (lactic acid), D-lactic acid, L-lactic acid and its racemates, beta-hydroxypropionic acid, alpha-hydroxyvaleric acid, beta-hydroxyvaleric acid, gamma-hydroxyvaleric acid, hydroxymalonic acid (tartronic acid), D-hydroxysuccinic acid (malic acid), L-hydroxysuccinic acid and their racemates, dihydroxysuccinic acid (tartaric acid) enantiomerically pure and their racemates (ravic acid), propenoic acid (acrylic acid), fumaric acid, maleic acid, citric acid, mesoxalic acid, acetonedicarboxylic acid, oxaloacetic acid, aconitic acid, tricarballylic acid, ascorbic acid, formalydehyde, glutaraldehyde, genipin, glucose, fructose, maltose, dextrose, and sucrose, and combinations thereof.

Further guidance of how to prepare the composite material according to the present disclosure can be obtained from the disclosed examples.

In context of the present disclosure, the platelet-derived extra-cellular vesicle (a) of the present disclosure is used in the context of therapeutic, diagnostic or cosmetic applications.

In some embodiments the cosmetic applications include promoting the growth of bone tissue. For example, promoting bone tissue growth might indirectly benefit certain cosmetic procedures or applications. For example, cosmetic applications include the field of maxillofacial surgery or reconstructive surgery.

In cases where individuals undergo maxillofacial surgery for corrective or cosmetic purposes (such as jaw realignment or chin augmentation), promoting bone tissue growth is advantageous for ensuring proper healing and long-term stability of the surgical outcome.

Some cosmetic procedures involve reshaping the facial structure, such as cheekbone augmentation or chin augmentation. In such cases, promoting bone tissue growth could be beneficial for achieving the desired contour and ensuring the longevity of the results.

Dental implants are considered for aesthetic reasons. Promoting bone tissue growth around dental implants is crucial for their success and longevity. Substances that encourage osseointegration (the fusion of the implant with the surrounding bone tissue) could indirectly benefit cosmetic dental procedures.

In some embodiments the platelet-derived extra-cellular vesicle (a) and/or the composite material according to the disclosure is used in medicine.

In some embodiments the platelet-derived extra-cellular vesicle (a) and/or the composite material according to the disclosure is used in a treatment selected from the group consisting of broken bones, bone fractures, bone loss, bone degradation, dental soft tissue degradation, dental bone diseases, periondontitis, alveolar bone resorption, dental implant placement, jaw bone infections associated with bone loss, osteomyelitis, osteonecrosis of the jaw (ONJ), osteoporosis, osteopenia, Paget's disease of bone, osteomalacia, rickets, hyperparathyroidism when associated with bone reduction, osteogenesis imperfecta (brittle bone disease), multiple myeloma when associated with bone reduction, metastatic bone disease (bone metastases), avascular necrosis (osteonecrosis), fibrous dysplasia, Gaucher's disease, hyperthyroidism when associated with bone reduction, renal osteodystrophy, anorexia nervosa when associated with bone reduction, Cushing's syndrome, rheumatoid arthritis when associated with bone reduction, psoriatic arthritis when associated with bone reduction, sarcoidosis when associated with bone reduction, systemic lupus erythematosus (SLE), thalassemia, hypophosphatasia when associated with bone reduction, Camurati-Engelmann disease, sickle cell disease when associated with bone reduction, chronic kidney disease when associated with bone reduction, marfan syndrome, Ehlers-Danlos syndrome (subtypes which are when associated with bone reduction), steroid-induced osteoporosis (resulting from long-term use of corticosteroids), inflammatory bowel disease (e.g., Crohn's disease, ulcerative colitis, which affect bone density), HIV/AIDS when associated with bone reduction, hemochromatosis when associated with bone reduction, post-polio syndrome affecting bones, diseases of dysfunction of bone metabolism, stimulation of osteoclasts, reduction of osteoblasts, migration/proliferation of (gingival) fibroblasts, and mastocytosis when associated with bone reduction, or combinations thereof.

Some embodiments also include a kit or a "kit of parts" comprising the platelet-derived extra-cellular vesicle (a) and/or the composite material according to the disclosure, further comprising at least one item selected from the group consisting of an applicator, a measuring cup, a mixing tool, a spatula, an instruction manual, a package leaflet, and a syringe, as well as any combination thereof.

In some embodiments the platelet-derived extracellular vesicle for use as part of a bone replacement and/or bone rebuilding material, being characterized by being positive for at least one biomarker selected from CD9, CD63, Syntenin, and TSG101, as well as combinations thereof.

In another embodiment the platelet-derived extracellular vesicle according to the disclosure is characterized by being additionally positive for at least one biomarker selected from the group consisting of CD41b, CD42a, CD42b, CD61, CD62P, von-Willebrand-factor, PDGF-BB, RANTES, and a protein of the ESCRT-complex, as well as a combination thereof, and/or being additionally positive for a growth factor selected from the group consisting of latent-transforming growth factor beta-binding protein, hepatocyte growth factor-like protein, and insulin-like growth factor-binding protein, as well as a combination thereof.

**Definitions**

The term "EV" or "EVs" is used as abbreviation for extracellular vesicle. In some embodiments the term "platelet-derived extracellular vesicle (a)" is used, which is equivalent to "platelet-derived EV" and "platelet-derived EVs according to the present disclosure".

The term "nucleic acid" as used herein generally comprises polyribonucleotides (RNA) and poly-deoxyribonucleotides (DNA), each in single-stranded and/or double-stranded form, linear or circular, or mixtures thereof, including hybrid molecules. RNAs include, without limitation, messenger RNAs (mRNA), non-coding (ncRNAs-including anti-sense-RNAs), silencer RNAs, microRNAs (miRNAs), short hairpin RNAs (shRNAs), small interfering RNAs (siRNAs), repeat-associated small interfering RNA (rasiRNA), piwi-interacting RNAs (piRNA), Y RNA, Long non-coding RNAs (long ncRNAs, lncRNA)), transfer RNAs (tRNA), ribosomal RNAs (rRNA), small nuclear RNA (snRNA), small nucleolar ribonucleic acid (snoRNA), spliced leader RNAs (SL RNA).

Short definitions of nucleic acid molecules disclosed herein:

- mRNA (Messenger RNA): RNA molecule that carries genetic information from DNA to the ribosome, where it serves as a template for protein synthesis.

- microRNA (miRNA): Small non-coding RNA molecules that regulate gene expression by binding to target mRNAs, leading to their degradation or inhibition of translation.

- DNA (Deoxyribonucleic Acid): Double-stranded molecule that contains the genetic instructions for the development, functioning, growth, and reproduction of all known living organisms.

- Structural RNAs: RNA molecules that provide structural stability to cellular components or complexes, such as ribosomal RNA (rRNA) and transfer RNA (tRNA).

- tRNA (Transfer RNA): RNA molecule responsible for carrying amino acids to the ribosome during protein synthesis.

- rRNA (Ribosomal RNA): RNA molecule that forms part of the ribosome and is involved in protein synthesis.

- Vault RNA: Non-coding RNA molecule found in cellular vaults, which are large ribonucle-oprotein complexes with suggested roles in transport and cellular signaling.

- Y RNA: Small non-coding RNA molecule that plays a role in DNA replication and RNA quality control processes.

- Small Interfering RNAs (siRNA): Double-stranded RNA molecules that interfere with the expression of specific genes by targeting complementary mRNA for degradation.

- Mitochondrial DNA: DNA found in the mitochondria, organelles responsible for energy production in eukaryotic cells. Mitochondrial DNA contains genes essential for mitochondrial function.

- Short DNA Sequences of Retrotransposons: DNA sequences derived from retrotransposons, which are genetic elements capable of moving from one location in the genome to another via an RNA intermediate. These sequences may include short repeats or remnants of retrotransposon activity.

The terms "polypeptide" and "protein" are used interchangeably herein. The term "polypeptide" refers to a protein or peptide that typically contains or consists of at least 20, and preferably at least 30, such as at least 50 amino acids. The term "peptide" refers to an oligomer containing or consisting of at least 2 amino acids to about 19 amino acids.

The term "being characterized by being positive" for at least one biomarker means that the respective biomarker can be detected in the extracellular vesicle with ELISA. In one embodiment the biomarker is selected from CD9, CD63, Syntenin, and TSG101.

Quantitatively determining a biomarker, particularly one found in extracellular vesicles (EVs), can be achieved by the following method:

1. Isolation of EVs, such as ultracentrifugation, Size Exclusion Chromatography (SEC), precipitation and/or antibody-based Isolation.

2. Biomarker Determination, such as by enzyme-linked Immunosorbent Assay (ELISA).

3. optionally, for analyzing the biomarkers, a known amount of a synthetic biomarker can be added to control the isolation and detection process and normalize the results.

Short definitions of "osteogenic protein(s)" disclosed herein:

- OP-1 (Osteogenic Protein-1): A bone morphogenetic protein (BMP) involved in bone formation and repair.

- OP-2: It refers to a specific isoform or variant of osteogenic protein, but without specific information, it's challenging to provide a concise definition.

- OP-3: Similar to OP-2, it denotes a specific isoform or variant of osteogenic protein.

- BMP2 (Bone Morphogenetic Protein 2): A protein that plays a crucial role in bone and cartilage development, as well as in the regulation of cell growth and differentiation.

- BMP3 (Bone Morphogenetic Protein 3): A protein involved in skeletal development and regulation of bone density.

- BMP4 (Bone Morphogenetic Protein 4): A protein important in embryonic development, specifically in mesoderm formation and patterning.

- BMP5 to BMP16: These are different isoforms or variants of bone morphogenetic proteins (BMPs) with varying roles in embryonic development, tissue differentiation, and organogenesis.

- IGF (Insulin-like Growth Factor): A protein that plays a key role in cell growth, differentiation, and development.

- TGF (Transforming Growth Factor): A group of proteins involved in various cellular functions such as proliferation, differentiation, and apoptosis.

- PDGF (Platelet-Derived Growth Factor): A protein involved in cell growth and division, particularly in blood vessel formation. PDGF has several isoforms, including PDGF-AA, PDGF-AB, and PDGF-BB, with PDGF-BB consisting of two identical B-chain subunits. PDGF-BB is a key factor in cellular regeneration and wound healing, while also being involved in pathological processes like tumor growth.

- GDF1 to GDF11 (Growth Differentiation Factors 1 to 11): Members of the transforming growth factor beta (TGF-$\beta$) superfamily, involved in various developmental processes including organogenesis and tissue differentiation.

The term "protein of the ESCRT-complex" refers to the "Endosomal Sorting Complex Required for Transport" which is a collection of protein complexes involved in membrane remodeling processes, particularly in endosomal sorting, membrane scission, and trafficking. The ESCRT machinery is made up of five major complexes, each with specific roles:

1. ESCRT-0, which recognizes and sequesters ubiquitinated cargo (proteins tagged for degradation) on endosomal membranes. Key proteins comprise Hrs (hepatocyte growth factor-regulated tyrosine kinase substrate), STAM (signal transducing adaptor molecule).

2. ESCRT-I, binds to ESCRT-0, further concentrates cargo, and initiates vesicle budding. Key proteins comprise Tsg101 (tumor susceptibility gene 101), VPS28, VPS37, and MVB12/UBAP1.

3. ESCRT-II assists in membrane deformation and invagination during the budding process. Key proteins comprise VPS22, VPS36, VPS25.

4. ESCRT-III drives membrane scission, the final step in the separation of vesicles from the membrane. Key proteins comprise charged proteins like CHMP4 (charged multivesicular body protein 4), CHMP2, CHMP3, and VPS2.

5. VPS4 Complex, which dissociates and recycles the ESCRT-III components after membrane scission using ATP hydrolysis. Key proteins comprise VPS4, VTA1.

Additional Regulatory Proteins which are associated with ESCRT-complex are ALIX and Bro1.

Thus, in one embodiment the protein of the ESCRT-complex is selected from the group consisting of Hrs, STAM, Tsg101, VPS28, VPS37, MVB12/UBAP1, VPS22, VPS36, VPS25, CHMP4, CHMP2, CHMP3, VPS2, VPS4, VTA1, Alix and Bro1, as well as combinations thereof.

"Alkaline bioglass" is defined herein as a type of material that contains significant amounts of alkali metals (such as sodium or potassium) along with other glass-forming components like silica ($SiO_2$), calcium oxide (CaO), and phosphorus pentoxide ($P_2O_5$). It is specifically designed to interact with biological environments, often used in medical applications for bone repair and tissue regeneration due to its ability to bond with bone and stimulate tissue growth.

To create "rounded particles" of bioglass, certain processing techniques can be applied to achieve the desired shape. Sintering after ball milling and plasma spheroidization are preferred in order to achieve rounded uniform particles.

The roundness of a particle is defined by its "circularity" (C) according to the formula $C = \dfrac{4\pi A}{P^2}$, wherein A is the area of the particle and P is the perimeter. A circularity value of 1 indicates a perfect circle. In one embodiment the rounded particles have a circularity of at least 0.80, at least 0.85, at least 0.90, at least 0.95, or of about 1.00. Rounded particles are typically more desirable in biomedical applications, as they exhibit better flow properties, reduced surface area, and less chance of causing inflammation.

Definition of conditions and/or diseases disclosed herein:

- Broken Bones: Refers to a fracture in the continuity of bone structure due to trauma or stress.

- Bone Fractures: Another term for broken bones, indicating a disruption in bone integrity.

- Bone Loss: Reduction in bone density or mass, often leading to weakened bones and increased risk of fractures.

- Bone Degradation: Refers to the deterioration or breakdown of bone tissue, leading to loss of bone density and strength.

- Dental Soft Tissue Degradation: Degeneration or damage to the soft tissues in the oral cavity, such as gums and mucosa.

- Dental Bone Diseases: Various conditions affecting the bone tissue in the jaw and surrounding areas, including infections, deformities, and degenerative diseases.

- Periodontitis: Inflammation and infection of the tissues surrounding and supporting the teeth, leading to bone loss and eventual tooth loss.

- Alveolar Bone Resorption: Loss of bone tissue in the tooth sockets (alveoli) due to various factors, often resulting in tooth instability.

- Dental Implant Placement: Surgical procedure to insert artificial tooth roots into the jawbone as a foundation for dental prosthetics.

- Jaw Bone Infections Associated with Bone Loss: Infections in the jawbone that lead to bone deterioration, often resulting from untreated dental infections or trauma.

- Osteomyelitis: Inflammation and infection of the bone, usually caused by bacteria.

- Osteonecrosis of the Jaw (ONJ): Death of bone tissue in the jaw, often associated with the use of certain medications, particularly bisphosphonates.

- Osteoporosis: Condition characterized by decreased bone density and increased risk of fractures.

- Osteopenia: Reduced bone density, considered a precursor to osteoporosis.

- Paget's Disease of Bone: Chronic disorder causing abnormal bone remodeling, leading to enlarged and weakened bones.

- Osteomalacia: Softening of the bones due to inadequate mineralization, typically caused by vitamin D deficiency.

- Rickets: Childhood bone disorder characterized by weakened and deformed bones, often due to vitamin D deficiency.

- Hyperparathyroidism: Overactivity of the parathyroid glands, leading to excessive levels of parathyroid hormone (PTH) and subsequent bone resorption.

- Osteogenesis Imperfecta (Brittle Bone Disease): Genetic disorder causing fragile bones prone to fractures.

- Multiple Myeloma: Cancer of plasma cells in the bone marrow, leading to bone destruction and increased fracture risk.

- Metastatic Bone Disease (Bone Metastases): Cancer that has spread to the bones from other primary sites, causing bone destruction.

- Avascular Necrosis (Osteonecrosis): Death of bone tissue due to interrupted blood supply.

- Fibrous Dysplasia: Bone disorder characterized by the replacement of normal bone with fibrous tissue, leading to weakened bones.

- Gaucher's Disease: Inherited metabolic disorder causing the accumulation of certain fatty substances in cells and organs, including bones.

- Hyperthyroidism: Overactive thyroid gland, which can lead to increased bone resorption and reduced bone density.

- Renal Osteodystrophy: Bone abnormalities associated with chronic kidney disease and disturbances in mineral metabolism.

- Anorexia Nervosa: Eating disorder characterized by severe calorie restriction, often leading to bone loss and osteoporosis.

- Cushing's Syndrome: Condition caused by prolonged exposure to high levels of cortisol, which can lead to osteoporosis among other symptoms.

- Rheumatoid Arthritis: Autoimmune disease causing inflammation of joints, which can lead to bone erosion and deformities.

- Psoriatic Arthritis: Inflammatory arthritis associated with psoriasis, leading to joint inflammation and bone damage.

- Sarcoidosis: Systemic inflammatory disease that can affect multiple organs, including bones, causing bone loss.

- Systemic Lupus Erythematosus (SLE): Autoimmune disease affecting various organs, including bones, leading to bone loss and osteoporosis.

- Thalassemia: Inherited blood disorder causing abnormal hemoglobin production, which can lead to bone marrow expansion and bone deformities.

- Hypophosphatasia: Rare genetic disorder resulting in abnormal bone mineralization and reduced bone density.

- Camurati-Engelmann Disease: Rare genetic disorder characterized by progressive thickening of the bones, leading to pain and muscle weakness.

- Sickle Cell Disease: Inherited blood disorder causing abnormal hemoglobin, which can lead to bone infarctions and bone loss.

- Chronic Kidney Disease: Progressive loss of kidney function, leading to disturbances in mineral metabolism and bone abnormalities.

- Marfan Syndrome: Genetic disorder affecting connective tissue, which can lead to skeletal abnormalities including bone overgrowth.

- Ehlers-Danlos Syndrome: Group of connective tissue disorders, some subtypes of which can affect bone integrity and lead to bone reduction.

- Steroid-Induced Osteoporosis: Bone loss resulting from long-term use of corticosteroid medications.

- Inflammatory Bowel Disease: Chronic inflammatory conditions of the digestive tract, such as Crohn's disease and ulcerative colitis, which can affect bone density.

- HIV/AIDS: Human immunodeficiency virus infection and acquired immune deficiency syndrome, which can lead to bone loss and osteoporosis.

- Hemochromatosis: Genetic disorder causing iron overload in the body, which can lead to bone loss and osteoporosis.

- Post-Polio Syndrome: Condition affecting individuals who have had polio, characterized by muscle weakness and, in some cases, bone abnormalities.

- Diseases of Dysfunction of Bone Metabolism: Group of disorders affecting the normal balance of bone formation and resorption, leading to bone abnormalities.

- Stimulation of Osteoclasts: Process that promotes the activity of cells responsible for bone resorption, leading to bone loss.

- Reduction of Osteoblasts: Decreased activity or number of cells responsible for bone formation, contributing to bone loss.

- Migration/Proliferation of (Gingival) Fibroblasts: Movement and growth of fibroblast cells, which can contribute to tissue remodeling and repair, including in the gums.

- Mastocytosis: Disorder characterized by the accumulation of mast cells in various tissues, which can lead to bone abnormalities and osteoporosis.

Important bone-formation pathways which are influenced by the EVs of the present disclosure:

- Wnt Signaling Pathway: Wnt signaling is essential for regulating bone formation (osteogenesis). Activation of the Wnt pathway promotes osteoblast differentiation and activity, thereby stimulating bone formation. Conversely, inhibition of Wnt signaling can lead to increased bone resorption by osteoclasts.

- Bone Morphogenetic Protein (BMP) Signaling Pathway: BMPs are members of the transforming growth factor-beta (TGF-beta) superfamily and play important roles in bone formation. BMP signaling promotes osteoblast differentiation and bone matrix synthesis, contributing to bone growth and repair.

- Notch Signaling Pathway: Notch signaling regulates various aspects of skeletal development and bone remodeling. It influences osteoblast and osteoclast differentiation, as well as the balance between bone formation and resorption.

- RANK/RANKL/OPG Signaling Pathway: Receptor activator of nuclear factor-kappa B (RANK), its ligand (RANKL), and osteoprotegerin (OPG) are key regulators of osteoclast differentiation and function. RANKL, produced by osteoblasts and other cells, binds to RANK on osteoclast precursors, leading to osteoclast formation and bone resorption. OPG acts as a decoy receptor for RANKL, thereby inhibiting osteoclastogenesis and bone resorption.

- MAPK (Mitogen-Activated Protein Kinase) Signaling Pathway: MAPK pathways, including ERK1/2, JNK, and p38, are involved in regulating various cellular processes in bone cells, including proliferation, differentiation, and survival. They play roles in both bone formation and resorption.

- TGF-beta (Transforming Growth Factor-beta) Signaling Pathway: TGF-beta signaling regulates osteoblast and osteoclast activities and is involved in bone remodeling. It can have both stimulatory and inhibitory effects on bone formation, depending on the context and the isoforms involved.

- Hedgehog Signaling Pathway: The Hedgehog pathway is involved in skeletal development and bone remodeling. It regulates osteoblast differentiation and bone formation, and dysregulation of Hedgehog signaling can lead to skeletal abnormalities.

- RANTES (Regulated upon Activation, Normal T Cell Expressed and Secreted), also known as CCL5 (C-C motif chemokine ligand 5), is a chemokine, which is a type of small signaling protein that plays a key role in the immune system. Specifically, RANTES belongs to the CC chemokine family and is involved in the regulation of immune cell migration and activation.

Embodiments

1. Platelet-derived extracellular vesicle (a) for use as bone replacement and/or bone rebuilding material.

2. The platelet-derived extracellular vesicle (a) according to embodiment 1, wherein the platelet-derived extracellular vesicle (a) has a diameter of between 100 and 200 nm.

3. The platelet-derived extracellular vesicle (a) according to any of embodiments 1 or 2, wherein the platelet-derived extracellular vesicle (a) is characterized by being positive for at least one biomarker selected from the group consisting of CD9, CD41b, CD42a, CD42b, CD61, PAC-1, CD62P, CD31, CD63, von-Willebrand-factor, Syntenin, TSG101, and a protein of the ESCRT-complex, as well as a combination thereof, and/or being positive for a growth factor selected from the group consisting of latent-transforming growth factor beta-binding protein, hepatocyte growth factor-like protein, and insulin-like growth factor-binding protein, as well as a combination thereof.

4. Composite material for use as bone replacement and/or bone rebuilding material, characterized in comprising the platelet-derived extracellular vesicles (a) according to any of embodiments 1 to 3 and further comprising at least one inorganic component and at least one organic component (c).

5. The platelet-derived extracellular vesicle (a) according to any of embodiments 1 to 3, or the composite material according to embodiment 4, for use as a stimulator of osteoblast activity and/or inhibitor of osteoclast activity.

6. The composite material according to embodiments 4 or 5, wherein the inorganic component, is a granulate made from a material selected from the group consisting of calcium phosphate, sodium-, potassium-, calcium- and/or silicate-containing acid, neutral and/or alkaline bioglass, and glass ceramic, and a combination thereof; wherein the granulate comprises biologically active, polygon-broken, rounded particles.

7. The composite material according to embodiment 6, wherein the calcium phosphate is selected from the group consisting of $\beta$-tricalcium phosphate, preferably $\beta$-tricalcium phosphate with 0 to 15% by weight of sodium magnesium silicate glass, monocalcium phosphate monohydrate, anhydrous monocalcium phosphate, dicalcium phosphate dihydrate, anhydrous dicalcium phosphate, tetra-calcium phosphate, whitlockite, octacalcium phosphate, hydroxyapatite, oxyapatite, type A carbonatapatite, type B carbonatapatite, calcium-deficient hydroxyapatite, amorphous calcium phosphate, amorphous carbonate-containing calcium phosphate, and calcium alkali orthophosphate glass ceramic, and a combination thereof.

8. The composite material according to any of embodiment 5 to 7, wherein the organic component is selected from the group consisting of a polymer, such as polypeptide, polysaccharide, and poly-lipid, and a combination thereof.

9. The composite material according to any of embodiments 5 to 8, wherein the organic component is selected from the group consisting of gelatine, collagen, glycosaminogly-can, hyaluronan, sodium hyaluronate, calcium hyaluronate, cellulose such as methyl-cellulose, ethylcellulose, propylcellulose, hydroxypropylcellulose, hydroxyethylcellulose, starch, dextran, hydroxyethyl starch, alginate, polyethylene glycol, albumin, and chitosan, or a combination thereof.

10. The composite material according to any of embodiments 5 to 9, wherein the organic component is selected from the group consisting of organically processed or demineralized allogeneic or xenogeneic bone material, synthetic polypeptide, parathyroid hormone, and osteogenic protein, or a combination thereof.

11. The composite material according to embodiment 10, wherein the osteogenic protein is selected from the group consisting of OP-1, OP-2, OP-3, BMP2, BMP3, BMP4, BMP5, BMP6, BMP-7, BMP9, BMP10, BMP11, BMP12, BMP14, BMP15, BMP16, IGF, TGF, PDGF, GDF1, GDF3, GDF5, GDF6, GDF7, GDF8, GDF9, GDF10, and GDF11, and a combination thereof.

12. The composite material according to embodiments 10 or 11, characterized by the inorganic component having at least one of the features selected from the group consisting of

being a ceramic component comprising calcium, phosphate, $SiO_2$, MgO and $Na_2O$,

being a granulate whose particles have a $d_{50}$ value of at least 10 $\mu$m,

being present in the composite material from 15 to 80 wt.-%, and

having an interconnecting, open multi-porosity with micro-, meso- and

macropores (pore diameter 0.05 $\mu$m - 500 $\mu$m) and a total porosity of from at least 35% up to 65%,

as well as combinations thereof.

13. The composite material according to embodiments 10 or 11, characterized by the inorganic component having at least one of the features selected from the group consisting of:

a) being present in the composite material with about 85 wt.-%, having a particle size from 1000-2000 $\mu$m, a total porosity of at least 65%, and a pore-size of from 0.1 to 500 $\mu$m;

b) being present in the composite material with about 80 wt.-%, having a particle size from 1000-2000 $\mu$m, a total porosity of at least 65%, and a pore-size of from 0.1 to 500 $\mu$m;

c) being present in the composite material with about 15 wt.-%, having a particle size from 150-500 $\mu$m, a total porosity of at least 65%, and a pore-size of from 0.1 to 300 $\mu$m; and

d) being present in the composite material with about 5 wt.-%, having a particle size from 150-500 $\mu$m, a total porosity of at least 35%, and a pore-size of from 0.1 to 30 $\mu$m.

14. Method for production of a composite comprising the steps of:

a. adding at least one organic component to water to form a suspension;

b. adding at least one inorganic component; and, optionally, a crosslinking agent; to the suspension of step a.) and mix the components;

c. transferring the solution from step b.) into a mold and then drying the mixture by lyophilization to form the composite material;

d. adding at least one platelet-derived extracellular vesicle (a) to water or buffer;

e. Immerse the composite material of step c.) with the solution of step d.).

15. The platelet-derived extracellular vesicle according to any of embodiments 1 to 4, or the composite material according to any of embodiments 5 - 13 for the use in medicine, such as in the treatment selected from the group consisting of broken bones, bone fractures, bone loss, bone degradation, dental soft tissue degradation, dental bone diseases, periondontitis, alveolar bone resorption, dental implant placement, jaw bone infections associated with bone loss, osteomyelitis, osteonecrosis of the jaw (ONJ), osteoporosis, osteopenia, Paget's disease of bone, osteomalacia, rickets, hyperparathyroidism when associated with bone reduction, osteogenesis imperfecta (brittle bone disease), multiple myeloma when associated with bone reduction, metastatic bone disease (bone metastases), avascular necrosis (osteonecrosis), fibrous dysplasia, Gaucher's disease, hyperthyroidism when associated with bone reduction, renal osteodystrophy, anorexia nervosa when associated with bone reduction, Cushing's syndrome, rheumatoid arthritis when associated with bone reduction, psoriatic arthritis when associated with bone reduction, sarcoidosis when associated with bone reduction, systemic lupus erythematosus (SLE), thalassemia, hypophosphatasia when associated with bone reduction, Camurati-Engelmann disease, sickle cell disease when associated with bone reduction, chronic kidney disease when associated with bone reduction, marfan syndrome, Ehlers-Danlos syndrome (subtypes which are when associated with bone reduction), steroid-induced osteoporosis (resulting from long-term use of corticosteroids), inflammatory bowel disease (e.g., Crohn's disease, ulcerative colitis, which affect bone density), HIV/AIDS when associated with bone reduction, hemochromatosis when associated with bone reduction, post-polio syndrome affecting bones, diseases of dysfunction of bone metabolism, stimulation of osteoclasts, reduction of osteoblasts, migration/proliferation of (gingival) fibroblasts, and mastocytosis when associated with bone reduction, or combinations thereof.

16. Kit comprising the platelet-derived extracellular vesicle according to any of embodiments 1 to 4, and/or the composite material according to any of embodiments 5 - 13, further comprising at least one item selected from the group consisting of an applicator, a measuring cup, a mixing tool, a spatula, an instruction manual, a package leaflet, and a syringe, as well as any combination thereof.

In one embodiment the composite material comprises:

- EVs characterized by being positive for at least one biomarker selected from CD9, CD63, Syntenin, and TSG101, as well as combinations thereof;

- An inorganic component selected from β-tricalciumphosphate (β-TCP), and

- An organic component selected from collagen.

In one embodiment the composite material comprises:

- EVs characterized by being positive for at least one biomarker selected from CD9, CD63, Syntenin, and TSG101, as well as combinations thereof;

- at least 65% (wt-%) of an inorganic component selected from β-tricalciumphosphate (β-TCP), and

- an organic component selected from collagen.

In one embodiment the composite material comprises:

- EVs characterized by being positive for at least one biomarker selected from CD9, CD63, Syntenin, and TSG101, as well as combinations thereof;

- an inorganic component selected from β-tricalciumphosphate (β-TCP), and

- an organic component selected from collagen and/or hyaluronic acid.

In one embodiment the composite material comprises:

- EVs characterized by being positive for at least one biomarker selected from CD9, CD63, Syntenin, and TSG101, as well as combinations thereof;

- an inorganic component selected from β-tricalciumphosphate (β-TCP) and and/or silica, and

- an organic component selected from collagen and/or hyaluronic acid.

In one embodiment the composite material comprises:

- EVs characterized by being positive for at least one biomarker selected from CD9, CD63, Syntenin, and TSG101, as well as combinations thereof;

- an inorganic component selected from β-tricalciumphosphate (β-TCP), having about 68 wt.% of granules with a particle size between 1000-2000 μm and about 17 wt.% of granules with a particle size between 150-500 μm, and/or

with an interconnected porosity of >= 65%,

- an organic component selected from collagen,

- and, optionally, one or more further additives.

In one embodiment the composite material comprises:

- EVs characterized by being positive for at least one biomarker selected from CD9, CD63, Syntenin, and TSG101, as well as combinations thereof;

- an inorganic component selected from β-tricalciumphosphate (β-TCP) and/or about 4 wt.% sodium- magnesium-silicate, having about 85 wt.% of granules with a particle size between 1000-2000 μm and/or an open porosity of >= 75%,

- an organic component selected from collagen,

- and, optionally, one or more further additives.

In one embodiment the composite material comprises:

- EVs characterized by being positive for at least one biomarker selected from CD9, CD63, Syntenin, and TSG101, as well as combinations thereof;

- an inorganic component selected from hydroxyapatite, having about 85 wt.% of granules with a particle size between 150-8000 μm and/or an open porosity of >= 80%,

- an organic component selected from collagen,

- and, optionally, one or more further additives.

In one embodiment the composite material comprises:

- EVs characterized by being positive for at least one biomarker selected from CD9, CD63, Syntenin, and TSG101, as well as combinations thereof;

- an inorganic component selected from an alkaline bioglass,

- an organic component selected from collagen,

- and, optionally, one or more further additives.

In one embodiment the composite material comprises:

- EVs characterized by being positive for at least one biomarker selected from CD9, CD63, Syntenin, and TSG101, as well as combinations thereof;

- an inorganic component selected from β-tricalciumphosphate (β-TCP), having a $d_{50}$ of 11,8-16,5μm, a maximum particle size of up to 63 μm,

- an organic component selected from sodium hyaluronate,

- and, optionally, one or more further additives, such as methyl-cellulose.

In one embodiment the composite material comprises:

- EVs characterized by being positive for at least one biomarker selected from CD9, CD63, Syntenin, and TSG101, as well as combinations thereof;

- an inorganic component selected from β-tricalciumphosphate (β-TCP), having an interconnected porosity of >= 65%,

- and, optionally, one or more further additives.

In one embodiment the composite material comprises:

- EVs characterized by being positive for at least one biomarker selected from CD9, CD63, Syntenin, and TSG101, as well as combinations thereof;

- an inorganic component selected from β-tricalciumphosphate (β-TCP) and/or about 4 wt.% sodium- magnesium-silicate, having an open porosity of >= 75%,

- and, optionally, one or more further additives.

In one embodiment the composite material comprises:

- EVs characterized by being positive for at least one biomarker selected from CD9, CD63, Syntenin, and TSG101, as well as combinations thereof;

- an inorganic component selected from hydroxyapathite, having an interconnected porosity of >= 80%,

- and, optionally, one or more further additives.

In one embodiment the composite material comprises:

- EVs characterized by being positive for at least one biomarker selected from CD9, CD63, Syntenin, and TSG101, as well as combinations thereof;

- an organic component selected from gelatine and/or collagen,

- and, optionally, one or more further additives.

Examples

## Example 1. Production of platelet-derived extracellular vesicles

Example 1.1 Platelet concentrate (PRP-PC)

450 ml of whole blood are collected in a 450-ml triple bag containing CPDA1 anticoagulant (TERUMO PENPOL, Ltd. Puliyarakonam, Trivandrum, India). Platelet rich plasma is separated from whole blood by light spin centrifugation by Heraeus 6000i, Germany refrigerated centrifuge at 1750 rpm for 11 minutes at 21°C, with acceleration and deceleration curves of 5 and 4 respectively and the platelets are concentrated by heavy spin centrifugation at 3940 rpm for 5 minute at 21°C, with acceleration and deceleration curves of 9 and 5 respectively with subsequent removal of supernatant plasma. The platelet concentrate bag is left stationary with the label side down at room temperature for approximately 1 hour.

The platelet poor plasma is frozen promptly and stored as fresh frozen plasma (FFP) at or below - 30°C for one year.

Example 1.2 Method for preparation of buffy coat-platelet concentrate (BC-PC)

450 ml of whole blood is collected in a 450-ml quadruple bag containing 63 ml of CPD anticoagulant, with additive solution (SAGM). (TERUMO PENPOL, Ltd.,Puliyarakonam, Trivandrum, India). The whole blood is first subjected to "hard spin" centrifugation at 3940 rpm for 5 minutes at 21°C with acceleration and deceleration curves of 9 and 4 respectively. Whole blood is separated into different components according to their specific gravity.

- The top layer - platelet poor supernatant plasma (150-200 ml).

- Middle layer - buffy coat, containing approximately 90% of platelets, 70% of WBCs and 10% of red cells.

- Bottom layer - packed red cells.

Platelet poor supernatant is expressed into one satellite bag and buffy coat into another satellite bag. About 20-30 ml of plasma is returned to buffy coat with the aim of cleaning the tubing from residual cells and obtaining an appropriate amount of plasma in the BC.

The SAGM solution is added to the red cells. The bags containing red cells and plasma are then removed. Red cells are placed at 4°C in cold room and platelet poor plasma into a -40°C deep freezer as fresh frozen plasma (FFP). The buffy coat is gently mixed with the plasma and again subjected to light spin" centrifugation at 1,100 rpm for 6 minutes at 21°C, with acceleration and deceleration curves of 5 and 4 respectively, along with one empty satellite bag. The supernatant, platelet-rich plasma is expressed into empty platelet storage bag and then tubing was sealed. The bag with residual WBCs and red cells was discarded.

Example 1.3 Single donor platelets (SDP)-apheresis-PC

The automated cell separator equipment is of intermittent flow or continuous flow cell technique, using single or double venous access. Continuous flow, double venous access, automated cell separator - CS3000 ® plus, Baxter, Fenwal division, Deerfield, 14, 60015, USA can be used.

1. Written consent of the donor is taken after explaining the procedure in detail, time taken and about possible hazards and benefits.

2. Venous access is an important consideration in apheresis donors and veins are examined at the time of selection because of the following reasons

- Long duration of procedure

- Prolonged flow rate

- Frequent need for two venipunctures with continuous flow equipment.

3. Age of the donors is documented.

4. Prior to the apheresis procedure, ABO / Rh typing and testing for infectious disease markers (HIV, HBV, HCV, VDRL and malaria) of platelet-apheresis-donors are done.

5. Donors who have taken aspirin or other NSAIDS, which are likely to affect the platelet function, are deferred.

6. Those donors who have platelet count > $1.5 \times 105/\mu l$ were taken for platelet-apheresis.

Example 1.4 Procedure

The procedure is performed in a closed system. A disposable kit is installed to the continuous flow separator, then the machine is primed. The donor is prepared by cleaning of two venipuncture sites at the antecubital area of both arms by betadine and spirit phlebotomy is done with minimal trauma to the donor. During the procedure, the blood is anti-coagulated at the point of withdrawal in a controlled manner, and the ratio of whole blood and anticoagulant (ACD) is maintained at 9:1 to 11:1. The anticoagulated blood is pumped into a spinning separation container. Red cells are packed by centrifugal force towards the outer edges of the container, and then the red cells exit the separation container. The lower density components, such as plasma, platelets, or WBCs are removed by plasma pump and enter the spinning collection container where platelets are packed by centrifugal force towards the outer edges of the container. The separated platelets remain packed in the container, while other constituents of blood are returned to the donor. At the end of the collection procedure, the platelet collection bag is shaken vigorously to detach the platelets from the wall of the bag and kept for 1 hour at room temperature to make it an even suspension. This whole procedure requires 1.5-2.5 hours. The final volume of the apheresis-PC ranged from 200-300 ml.

Example 1.5 Production of human thrombocyte lysates (hPL)

Production of human thrombocyte lysate (= human platelet lysate (hPL)) is based on further processing of expired human thrombocyte concentrates (= human platelet concentrates (hPC/hTC). To gain hTCs usually two different proceeding methods are used. One is defined by the use of pooled buffy coat products deriving from whole blood donations of several donors, in the other apherese-concentrates are used. In this technique a donor is connected to an extra-corporal cell-separator and thrombocytes are filtered out, while other blood components like erythrocytes, leucocytes and plasma are given back to the donor. In both cases the resulting blood products are leucocyte-depleted TCs. These products contain living thrombocytes for at least 4-5 days and can be used e.g. to replace lacking thrombocytes in patients with thrombocy-topenia.

After date of expiry, excess products can be used for production of hPL. By freezing and thawing-cycles (-20 °C and RT) platelets are broken up/cracked and release their inner contents into the surrounding liquid (plasma). Different protocols exist including centrifugation steps between 3200 and 10 000 g for about 1 hour. Lysed cells, cellular fragments and other debris is/are depleted by centrifugation. The result is a clear, vicious, yellow liquid, consisting of the platelet lysates and plasma. To sterilize the product after proceeding, 200 nm filters can be used optionally in addition. In parallel particles bigger than 200 nm are removed, including extra-cellular vesicles, which are not at the size of exosomes.

**Example 2. Production of composite material**

The composite materials according to the present disclosure comprises components A, B, C, D, E, F, G, H, I, K, L, M, N, O, P, Q, R, and S.

The components are as follows:

Component A: 99% pure β-tricalcium phosphate, particulate, with a bulk density of $1.1 \pm 0.1$ g/cm$^3$ and a particle size of <63 $\mu$m ($d_{50}= 15 \pm 5$ $\mu$m).

Component B: Bioresorbable sintered glass consisting of $75 \pm 5$ wt.% SiO2, $9 \pm 3$ wt.% MgO, and $15 \pm 5$ wt.% $Na_2O$.

Component C: Rapidly resorbable glass ceramic with the chemical formula $Ca_2KNa(PO_4)_3$ and the following elemental composition: $23 \pm 2$ wt.% Ca, $11.5 \pm 2$ wt.% K, $6.5 \pm 2$ wt.% Na, $55 \pm 2$ wt.% $PO_4$, and $2.5 \pm 1$ wt.% Mg.

Component D: Water for injection purposes.

Component E: Ammonium bicarbonate with a particle size of <10 $\mu$m.

Component F: Ammonium bicarbonate with a particle size of 10-50 $\mu$m.

Component G: Ammonium bicarbonate with a particle size of 50-500 $\mu$m.

Component H: Component A, sprayed intermittently with component D on a granulation plate under rolling motion to induce rolling according to a snowball effect. After a 5-hour sintering process at 1000°C followed by sieving, spherical granules ranging from 150-5000 $\mu$m with approximately 30-40% porosity and a density of about 1.2 g/cm$^3$ are obtained. X-ray powder diffraction confirms pure β-tricalcium phosphate. Specific surface area measured using the BET method with krypton as the measurement gas yields values in the range of 0.1-0.15 m$^2$/g.

Component I: Component A is intimately mixed with 10 wt.% component E, 20 wt.% component F, and 10 wt.% component G, then cold isostatically pressed. After a sublimation process at 80°C for 12 hours followed by sintering at 1000°C for 5 hours and subsequent crushing and sieving, granules sized 150-8000 $\mu$m are obtained with approximately 65% porosity, a bulk density of 0.8 g/cm$^3$, and confirmed phase purity by X-ray powder diffraction. The pore distribution is discrete, with only the micropores (<10 $\mu$m) interconnected. Mesopores (10-50 $\mu$m) and macropores (>50 $\mu$m) are discretely distributed within the interconnected microporous network and are accessible to blood and body fluids. The BET method using krypton as the measurement gas yields a specific surface area of 0.15-0.2 m$^2$/g.

Component K: Reticulated polyurethane foams, produced according to the Schwartzwalder-Somers method, with a pore size distribution of 45 pores per inch (ppi, corresponding to a pore size of 1000 $\mu$m).

Component L: Reticulated polyurethane foams, produced according to the Schwartzwalder-Somers method, with a pore size distribution of 80 pores per inch (ppi, corresponding to a pore size of 500 $\mu$m).

Component M: Component A is slurred with component D, 5 wt.% component B is added, and finely ground to achieve a thixotropic slurry with a particle size distribution of 2-5 $\mu$m ($d_{50}$). This slurry is kneaded into components K and L. After sintering at 1000°C for 5 hours, the PU foam is completely burned out, leaving behind a firmly sintered ceramic with pore sizes of 400 $\mu$m (initial component K) or 250 $\mu$m (initial component L). After crushing, sieving, and repeated sintering under the same conditions, granules of sizes 150-8000 $\mu$m are obtained with a total porosity of 80%, a bulk density of about 0.6 g/cm$^3$, and each half with the mentioned pore diameters. X-ray powder diffraction confirms pure β-tricalcium phosphate.

Component N: Component C is slurred with component D, 5 wt.% component B is added, and finely ground to achieve a thixotropic slurry with a particle size distribution of 2-5 $\mu$m ($d_{50}$). This slurry is kneaded into components K and L. After sintering at 1000°C for 5 hours, the PU foam is completely burned out, leaving behind a firmly sintered ceramic with pore sizes of 400 $\mu$m (initial component K) or 250 $\mu$m (initial component L). After crushing, sieving, and repeated sintering under the same conditions, granules of sizes 150-8000 $\mu$m are obtained with a total porosity of 80%, a bulk density of about 0.6 g/cm$^3$, and each half with the mentioned pore diameters. X-ray powder diffraction confirms membership in phase KNaCa2(PO4)2 (PDF #51-579).

Component O: Porcine collagen, mainly type 1, obtained from tissue of controlled grown pigs. The material is declared as "released for human consumption." The porcine tissues are processed under controlled environmental conditions. The collagen is highly purified, with residues of the manufacturing process present only in traces, ensuring the absence of toxic effects.

Component P: Acetic acid 100%.

Component Q: Porcine gelatine obtained from tissue of controlled grown pigs. The material is declared as "released for human consumption." The porcine tissues are processed under controlled environmental conditions.

Component R: Genipin.

Component S: Platelet-derived extracellular vesicle (a) of at least 5 vol.-% in a suitable buffer, such as PBS (pH 7.4).

The particle fraction 1000-2000 $\mu$m of component I was sieved and 5.6 g of each were suspended in 20 ml of component D, with 74 $\mu$l of component P added. After 0, 1, 3, 6, and 23 hours, the material was filtered, washed, and dried. Subsequently, the particles were washed, filtered, dried, and subjected to particle size analysis using a laser scattering device (Fritsch Analysette), scanning electron microscopy to assess surface integrity, and X-ray powder diffraction for phase purity. Table 1 shows $d_{10}$, $d_{50}$, and $d_{90}$ values of tricalcium phosphate powder after various durations of exposure to acetic acid.

Table 1

| [h] in acidic acid | $d_{10}$ [$\mu$m] | $d_{50}$ [$\mu$m] | $d_{90}$ [$\mu$m] |
|---|---|---|---|
| 0 | 12.6 | 30.09 | 51.74 |
| 1 | 11.76 | 30.65 | 50.05 |
| 3 | 10.52 | 25.16 | 43.38 |
| 6 | 8.74 | 24.06 | 42.31 |
| 23 | 9.79 | 21.98 | 37.27 |

As shown in Table 1, a significant reduction in particle size [$\mu$m] occurs only after 6 hours, with the fine fraction represented by the $d_{10}$ value undergoing more pronounced degradation than the particulate breakdown of coarser fractions. Thus, the surface etching of small particles with larger surface areas is considered to have a stronger influence than the grain boundary etching. Even after 23 hours, the fine fraction has not significantly crossed the critical threshold of the phagocytizable range, indicating that an increased risk of excessive foreign body reactions due to implantation of component I into bone or tissue is also excluded after prolonged acid treatment.

The $d_{50}$ (also known as the median particle size) is a statistical measure used in particle size distribution analysis. It represents the particle size at which 50% of the sample, by mass or volume, is composed of particles smaller than this size, and 50% is composed of particles larger than this size. The same applies to "$d_{10}$" and "$d_{90}$", referring to "10% smaller" or "90% smaller", respectively. $d_{50}$ is therefore the particle diameter at which half of the particles in a sample are smaller and half are larger. For example, if a sample has a $d_{50}$ value of 10 micrometers ($\mu$m), this means that 50% of the sample's particles are smaller than 10 $\mu$m and 50% are larger than 10 $\mu$m.

From X-ray powder diffraction phase purity can be controlled. The exposure to acetic acid also has no effect on phase purity, thus excluding both an increased risk of excessive foreign body reactions due to implantation of component I into bone or tissue and a reduction in resorbability due to conversion into a non-resorbable calcium phosphate phase after prolonged acid exposure.

16.5 g of component O are added to 3.5 ml of component D and suspended using a colloid mill. The particle fraction 1000-2000 $\mu$m of component I is sieved, and 5.6 g are added to the suspension. After the addition of 74 $\mu$l of component P, the material is stirred at room temperature. After 0, 30, 120, and 165 minutes, the acid is neutralized, the mixture is cast into shape, and freeze-dried. X-ray powder diffraction is used to determine whether the acid exposure time had any effect on the phase composition of the calcium phosphate. The results show that the acid exposure had no effect on the phase composition of the material.

From component H, the granule fraction 150-500 $\mu$m is sieved out. From component I, the fractions 150-500 $\mu$m and 1000-2000 $\mu$m are sieved out. These components are mixed in various ratios according to Table 2. 190 g of component O is suspended in 1000 ml of component D and ground using the colloid mill. 760 g of the granule mixture is added, and the mixture is stirred thoroughly. After the addition of 10 ml of component B, the mixture is neutralized, poured into a square stainless-steel mold, and lyophilized. Depending on the lyophilization procedure (slow or fast), composite materials with densities of 0.2 or 0.4 g/ml can be achieved. Table 2 provides information on the various mixtures and the measured densities and specific surface areas.

**Table 2**

| Mixture | Component H 150-500 $\mu$m [wt.-%] | Component I 150-500 $\mu$m [wt.-%] | Component I 1000-2000 $\mu$m [wt.-%] | Collagen [wt.-%] | Density [g/ml] | Specific Surface [m2/g] | Ratio density to surface |
|---|---|---|---|---|---|---|---|
| CF-33 | 4 | 4 | 72 | 20 | 0.4 | 0.2605 | 1.54 |
| CF-34 | 2 | 6 | 72 | 20 | 0.4 | 0.25 | 1.6 |
| CF-35 | 4 | 12 | 64 | 20 | 0.4 | 0.2199 | 1.82 |
| CF-36 | 0 | 20 | 60 | 20 | 0.4 | 0.2152 | 1.86 |
| CF-37 | 4 | 4 | 72 | 20 | 0.2 | 0.3658 | 0.32 |
| CF-38 | 2 | 6 | 72 | 20 | 0.2 | 0.3092 | 0.65 |
| CF-39 | 4 | 12 | 64 | 20 | 0.2 | 0.306 | 0.66 |
| CF.40 | 0 | 20 | 60 | 20 | 0.2 | 0.3547 | 0.56 |
| Collagen | 0 | 0 | 0 | 100 | 0.3 | 1.5758 | 0.19 |

It is evident that 100% pure collagen exhibits a surface area 5 to 8 times larger. Consequently, the porosity is significantly reduced. Initially, the pores of the ceramic are not accessible because the collagen has penetrated into them and pervaded the porous ceramic structure. By adding particulate calcium phosphates, the ratio between density and specific surface area can be adjusted over wide ranges.

Variations of composite material of the present application

**Variant 1**

2.25g of component Q are mixed with 15ml of component D and heated with stirring to 70°C. After complete dissolution of component Q, and cooling the solution to 40°C, first 0.0225g of component R and then 13.5g of the granulate fraction 150-500 $\mu$m of component I as well as 1g of the fraction <150 $\mu$m of the same substance are added. The still warm solution is then poured into a mold and placed in the refrigerator to cool and cross-link. Demolding of the implants can be performed after 12 hours. Sterilization can be carried out by gamma rays.

**Variant 2**

1.5g of component Q are mixed with 15ml of component D and heated with stirring to 70°C. After complete dissolution of component A, 0.0225g of component R, 20g of the granulate fraction <150 $\mu$m of component I, and 2.5g of the granulate fraction 150-500 $\mu$m of component I are added. Air is then blown into the solution and the foamed material is poured into a mold. The mold is placed in the refrigerator to cool and cross-link. Demolding of the implants can be performed after 12 hours. Sterilization can be carried out by gamma rays.

**Variant 3**

From component M, the granulate fraction 150-500 $\mu$m as well as the fraction 1000-4000 $\mu$m are sieved out and mixed in a ratio of 1:1. 190g of component O is suspended in 1000ml of component D and milled using the colloid mill. The mixture of the two granule fractions is added, and the mixture is stirred thoroughly. After adding 10ml of component B, the mixture is neutralized, poured into a square stainless-steel mold, and lyophilized. Depending on the lyophilization process (slow or fast), composite materials with densities of 0.2 or 0.4 g/ml can be achieved. Sterilization can be carried out by gamma rays.

**Variant 4**

From component M, the granulate fraction 150-500 $\mu$m as well as the fraction 1000-4000 $\mu$m are sieved out and mixed in a ratio of 1:1. 190g of component O is suspended in 1000ml of component D and milled using the colloid mill. The mixture of the two granule fractions is added, and the mixture is stirred thoroughly. After adding 10ml of component B, the mixture is neutralized, poured into a square stainless-steel mold, and lyophilized. Depending on the lyophilization process (slow or fast), composite materials with densities of 0.2 or 0.4 g/ml can be achieved.
Sterilization can be carried out by gamma rays.
Component S can be added to the composite material after sterilization. Component S is suspended in a suitable buffer, such as phosphate buffered saline (PBS) and added to the composite material to a final EV concentration from $1 \times 10^8$ to $1 \times 10^9$, for example $3.75 \times 10^8$ particles/mL.
The final composite material is packaged and stored at 4°C for further use.

**Example 3. Administration of composite material and/or EVs**

The composite material according to the present disclosure is administered intra-operatively (during surgery) into the defect location or, in case of EVs only, systematically such as intravenous.
The composite material is soaked with saline solution or other autologous liquids/materials (like blood) prior to administration. For application of the composite material and/or the EVs, the application kit as disclosed hereinunder is used.

**Example 4. Characterization of cells with EVs according to the present disclosure and the composite material according to the present disclosure**

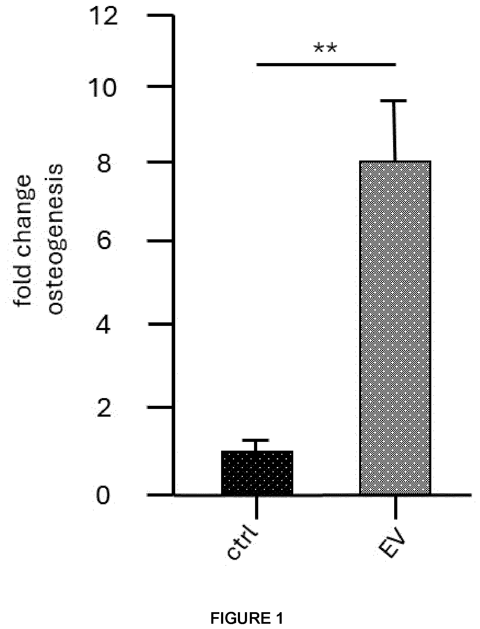

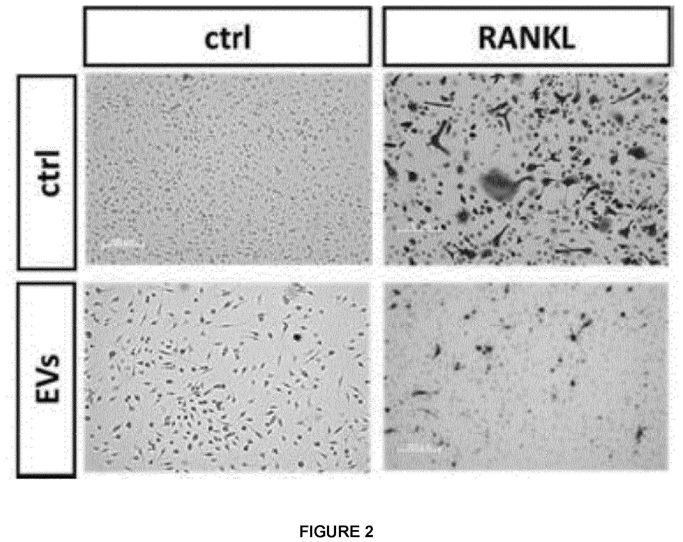

Please also refer to Figures 1-20 and their respective figure descriptions for additional experimental results. To measure bone regeneration potential of platelet-derived EVs, gingival primary fibroblast (gingival, primary cells from murine and human species), osteoblasts and osteoclasts were examined.
hPLEV-F were analysed for their content in relation to an EV-F of pooled human plasma. All samples were subjected to EV-lysis using an ultrasonic ice water bath at 35 kHz for 3x10min. Protein detection was performed using LEGENDplex™ Human Bone Formation Panel (5-plex) w/ VbP V02 (Biolegend) according to manufacturer's protocol. OPG, PDGF-BB, ALPL, Leptin and BMP-2 protein levels were determined using multiplex (Thermo-Fisher Scientific). Target levels were analysed using Novocyte Advanteo flow cytometry (Agilent). Additionally, RANTES (CCL5) protein detection was performed using ELISA MAX™ Deluxe Set Human CCL5 (RANTES) (Biolegend). Secreted RANTES levels were measured using the endpoint method, spiral average (12 points) at an absorbance of 450 nm (Spectrostar Nano, BMG).

Example 4.1 - Fibroblast scratch assay

Fibroblast (fibroblast scratch assay) represent a model for wound regeneration; osteoblast activity represents a model for bone regeneration and osteoclast activity represents a model for bone degeneration.
The cell assays were stimulated with tissue necrosis factor-$\alpha$ (TNF-$\alpha$) as it is considered to strongly influence degenerative processes in osteogenesis and mimics inflammatory activities.
We stimulated the gingival primary fibroblasts (gingival, primary cells from murine and human species) in-vitro and analyzed their metabolic activity, growth, survival and expression of osteogenic markers and calcium deposits. Specific osteogenetic marker were analysed in quantitative expression analysis. Osteoclasts and osteoblasts were stimulated with platelet-derived extracellular vesicles according to the present disclosure.
The metabolic activity of the cells was assessed via colorimetric MTT assay. Cells with an active metabolism convert MTT to violet formazan, which is commonly used to analyse relative cell numbers.

The term "colorimetric MTT" refers to a biochemical assay that measures cell viability or proliferation using the compound MTT (3-(4,5-dimethylthiazol-2-yl)-2,5-diphenyltetrazolium bromide). MTT is a yellow tetrazolium salt that is converted into purple formazan crystals by metabolically active cells.

Important steps of colorimetric MTT assay:

- Cells of interest are typically grown in culture dishes or plates under appropriate conditions.

- Cells are treated with various compounds, drugs, or conditions to assess their effects on cell viability or proliferation.

- After the desired treatment period, MTT solution is added to the cells. MTT is taken up by cells and converted by mitochondrial enzymes into insoluble formazan crystals.

- Following a specified incubation period, the culture medium is removed, and a solvent such as dimethyl sulfoxide (DMSO) is added to dissolve the formazan crystals.

- The resulting solution is then assessed using a spectrophotometer, which measures the absorbance of the formazan dye at a specific wavelength. The intensity of the colour is directly proportional to the number of metabolically active cells present in the culture.

For further investigation of growth characteristics of gingival fibroblasts, we performed a scratch assay on complete monolayers of gFB grown for one week in DMEM (cf. also figure 11).

The fibroblast scratch assay, also known as the wound healing assay or the cell migration assay, is a widely used experimental technique to study cell migration and wound healing processes in vitro. It involves creating a "scratch" or artificial wound in a confluent monolayer of cultured fibroblast cells and observing the migration of these cells into the denuded area over time. The general description of the procedure involves the following steps:

- Fibroblast cells are cultured in appropriate cell culture vessels such as petri dishes or multi-well plates until they reach confluence, forming a monolayer. The cells are typically grown in a suitable growth medium under standard cell culture conditions.

- Once the cells have formed a confluent monolayer, a "scratch" or wound is made in the cell monolayer using a fine tool such as a pipette tip, a needle, or a specialized scratching device. This creates a gap or denuded area in the cell layer.

- After making the scratch, the culture medium is carefully aspirated or removed to eliminate detached cells and debris. The dish or plate is then gently washed with a buffer solution to further remove any residual debris.

- Fresh culture medium is added to the dish or plate to provide nutrients and support cell migration into the scratch area.

- The dish or plate is placed under a microscope equipped with a camera to allow for real-time or time-lapse imaging of cell migration. Images are typically captured at regular intervals over a specified period to monitor the progression of cell migration into the scratch area.

- The extent of cell migration and wound closure can be quantified by measuring parameters such as the percentage of scratch closure, the migration rate of cells, and the width of the scratch over time. Various image analysis software packages are available to assist with quantitative analysis.

In summary these data show a significant increase in cell growth for EV stimulated fibroblasts, affecting the wound healing capacity.

Example 4.2 - Osteoblast cultures

*Example 4.2.1 - Isolation and cultivation of osteoblast cultures*

Primary osteoblasts were isolated from long bones of the fore and hind limbs of 4 to 5-week-old C57Bl6 mice. After the removal of bone marrow, the bones were washed in PBS and cut into small pieces prior to collagenase digestion (500 U/ml Collagenase II in DMEM) for 2 h at 37°C. Bone fragments were washed three times in proliferation medium (DMEM low glucose (Thermo-Fisher Scientific), 100 U/ml penicillin, 100 μg/ml streptomycin (Thermo-Fisher Scientific),

50 μg/ml gentamycin (Sigma-Aldrich), and 1.25 μg/ml Fungizone (Sigma-Aldrich)) and transferred to a T25-flask with 5 ml proliferation medium. The medium was changed for the first time after 5 days of incubation. Subsequent media changes were performed three times a week until cells reached confluency.

*Example 4.2.2 - Differentiation*

Cells were seeded in a density of $2.8*10^4$ cells per well on 24-well plates. After 2 days in culture, cells reached approximately 80% confluency and the proliferation medium was exchanged for differentiation medium (proliferation medium supplemented with 100 μg/ml L-ascorbic acid, 10 nM β-glycerol phosphate, and 100 nM dexamethasone).

*Example 4.2.3 - Osteoclast cultures*

Osteoclasts were generated from the bone marrow of 4-week-old C57Bl6 mice. As described previously (Doding, Hufner, et al., 2023) bone marrow cells (BMC) were centrifuged out of the long bones of the front and hind limbs (10,000 × g for 20s). BMC were resuspended in proliferation medium (αMEM containing 10% FBS (Gibco) and 1% penicillin/streptomycin) and incubated overnight in 10 ml proliferation medium on a 10 cm dish (one per animal). For treatment with hPLEV-F, non-adherent cells were seeded in a density of $3*10^5$ cells per well in 600 μl proliferation medium 50 ng/ml M-CSF (PeproTech) in 24-well plates on round coverslips (Menzel™Mi-croscopic Coverslips round 8mm, Thermo Fisher Scientific).

*Example 4.2.4 - Osteoblast proliferation activity*

Osteoblast proliferation activity was determined using a crystal violet assay (Sigma-Aldrich) according to the manufacturer's recommendations. Briefly, cells were seeded in 24-well plates and proliferated for 3 days in proliferation medium as described previously (Doding, Zimmermann, et al., 2023). After 24h, hPLEV-F were added before including an inflammatory trigger (TNFα) after another 12h of incubation. After 3 days, cells were washed with PBS and stained using 100μl of a crystal violet solution (0.5g crystal-violet-powder, 80ml distilled $H_2O$, 20ml Methanol) for 15 minutes. Cells were washed three times with $ddH_2O$, cells and photographed using a camera (EOS 77D, Canon), mounted on an inversed microscope (Primovert iLED Microscope, Carl Zeiss). Afterwards, plates were dried for 4h at 37°C until the dye was dissolved using 120 μl/well methanol. Samples were evaluated using a photometric Infinite M Nano plate reader (Tecan Life Science) at an absorption of 595nm. Each sample was double evaluated in a 48 well plate. Proliferation was expressed using a proliferation index by calculating the crystal violet absorption intensity normalized to the control group.

*Example 4.2.5 - Osteoblast mineralization activity*

Osteoblast mineralization activity was determined using an alizarin red assay (Sigma-Aldrich) according to the manufacturer's recommendations. Briefly, at day 14, cells were fixed for 15 minutes in 10 v/v-% PF and incubated with 40 mM alizarin red for 20 minutes. Free alizarin red was washed away with water prior to cell lysis using 10% acetic acid for 30 minutes. Afterwards, the suspension was heated to 85°C for 10 min and allowed to cool down for 5 min on ice. Then, samples were centrifuged for 15 min at 20,000 × g and the pH of the supernatant was neutralized with 10% ammonium hydroxide. Values were determined at OD405 (Infinite M Nano plate reader, Tecan Life Science). Concentrations were determined by comparing values to a standard curve and normalized to an untreated control. The experiment was repeated three times with two wells per condition. Wells were photographed using a camera (EOS 77D, Canon) mounted on an inversed microscope (Primovert iLED Microscope, Carl Zeiss).

*Example 4.2.6 - Immunofluorescence staining of osteoblasts*

Osteoblasts were seeded in a concentration of $1.4 \times 10^4$ cells on rat-tail collagen Type I (Sigma-Aldrich)-coated coverslips under hPLEV-F-treatment and in differentiation medium. After 14 days of treatment, with medium change every 2-3 days, cells were fixed in 4 v/v-% PFA for 5 min, washed with PBS three times, prior to preconditioning using 1.5% normal-goat serum (NGS) + 0.1 wt-% TX-100 (Triton). Then the anti-osteocalcin-antibody (rabbit ab93876, Abcam, 1:500 in 1.5 wt-% NGS) was applied overnight at 4°C. After 3 washing cycles using PBS the secondary antibody was applied (Alexa Fluor 488 anti-rabbit IgG (H+L), Invitrogen, 1:1,000 in 1.5 wt-% NGS). Thereafter, nuclei were stained with DAPI (Thermo Fisher Scientific; 1:10,000 in PBS). After washing the excess dye using 3x PBS and 1x $ddH_2O$, coverslips were mounted on slides using Mowiol (Mowiol 4-88, Carl Roth).

*Example 4.2.7 - Osteoblast differentiation*

The osteoblast differentiation in-vitro was characterized in three stages:

1) Cell Proliferation

Different Osteoblast Differentiation in-vitro assays were examined. Progenitor stem cells are seeded in monolayer on treated tissue culture plastic cell culture dishes or plates, or on biomaterial substrates, and allowed to proliferate in proliferation medium until they reach confluency with bi- or tri-weekly medium changes. After confluency is reached, cell culture medium is then changed from proliferation medium to differentiation medium that contains supplements to promote the osteogeneic differentiation of the progenitor cells and the formation of a mineralized matrix. The cells are cultured for up to 28 days in total with medium changes bi-weekly or every other day.

2) Cell Mineralization

To assess the extent of differentiation of the progenitor cells after the osteoblast differentiation assay, the calcium deposits are quantified using the non-destructive fluorescent image analysis as described in ASTM F3106 (published 2022 by FDA as "CBER Standards Recognition Program for Regenerative Medicine Therapies Standard Recognition Summary (SRS)") or terminated and measured directly by conducting a total calcium content analysis using one of the many commercial colorimetric kits available for this purpose.

Generally, osteocalcin staining is used to visualize and study osteocalcin, a protein predominantly found in bone tissue. Osteocalcin is secreted by osteoblasts, the cells responsible for bone formation, and is involved in the mineralization of bone.

The staining procedure typically involves the following steps:

- Preparation of Samples: Tissue samples containing bone or cells derived from bone are fixed using appropriate fixatives such as formaldehyde to preserve their structure.

- Embedding: If necessary, the fixed samples are embedded in a suitable embedding medium such as paraffin wax or cryo-sectioning medium to facilitate sectioning.

- Sectioning: The embedded samples are cut into thin sections using a microtome. The thickness of the sections depends on the specific requirements of the experiment.

- Deparaffinization (if applicable): If paraffin embedding is used, sections are de-paraffinized to remove the embedding medium and rehydrated before staining.

- Staining: The sections are incubated with a specific antibody against osteocalcin. Immunohistochemistry (IHC) or immunofluorescence (IF) techniques are commonly used for osteocalcin staining. The antibody binds specifically to osteocalcin present in the tissue sections.

- Visualization: In immunohistochemistry, a chromogenic substrate is added, which produces a colored reaction product at the site of antibody binding. This allows visualization of osteocalcin distribution under a microscope. In immunofluorescence, a fluorophore-conjugated secondary antibody is used to detect the primary antibody, leading to fluorescent signal emission upon excitation.

- Counterstaining (optional): To visualize cellular morphology and provide contrast to the staining, sections can be counterstained with dyes such as hematoxylin or DAPI (4',6-diamidino-2-phenylindole).

- Mounting and Imaging: Once stained, the sections are typically mounted onto glass slides using mounting media to preserve the staining. They are then examined under a microscope, and images are captured to document the distribution and intensity of osteocalcin staining.

3) Quantification of expressed osteogenic genes or proteins

Quantifying the expression of osteogenic genes or proteins is another important measurement to use in conjunction with measurement of calcified deposits to confirm the presence of osteoblasts. Both gene and protein measurements should be performed at multiple time points, as many peak and then later decrease. Here we investigated RANK, RANKL, TRAP, OPG, Col1A1, TNFalpha, RUNX2, OCN.

One potential protocol for Osteoclast differentiation: Osteoclast differentiation was examined using a tartrate-resistant acid phosphatase (TRAP) staining. Selected wells were treated with hPLEV-F after seeding. After 24h, a differentiation stimulus was applied (TNFα or RANKL) and cells differentiated for 5 days. Cells were fixed using 10 wt-% FBS for 3 min and equal volumes of ethanol and acetone. After drying, cells were stained as described before (Doding, Hufner, et al., 2023) and washed with PBS. Coverslips were mounted to microscopy slides with Mowiol (Mowiol 4-88, Carl Roth) and visual analysis was performed using light microscopy (Jenaval, Carl Zeiss) and a camera (AxioCam MRc5, Carl Zeiss).

Example 4.2.8 - Osteoclastic resorption

A functional analysis of osteoclastic resorptive activity was performed using the Bone Resorption Assay Kit (Cosmo Bio Co. LTD) according to the manufacturer's protocol. Briefly, cells were seeded in a concentration of $2,0 \times 10^5$ cells per well in $500\mu l$ phenol red free medium (Gibco MEM alpha (w/o Phenol red) + 10% FBS + 100 U/ml penicillin, 100 $\mu$g/ml streptomycin (Thermo-Fisher Scientific) + 1% M-CSF (Peprotech) in a 48 well plate with a specific layer of calcium phosphate combined with a fluoresceinamine-labeled chondroitin sulphate (FACS) coating. Activated osteoclasts dissolve the calcium phosphate and the FACS-layer is released into the supernatants. The fluorescence was measured using a Tecan infinite 200Pro (Tecan Life Science) at excitation wavelength of 485nm and emission wavelength of 527nm. The experiment was repeated four times with two wells per condition.

Example 4.2.9 - Osteoclast activity

Tartrate-resistant acid phosphatase (TRAP) staining and pit Assays:
TRAP staining of adherent cultures was done with a kit. The stained cells developed red color of different intensity. For measurement of resorbed area in the pit assay, the bovine cortical bone or dentin slices were washed with PBS, incubated in sodium hypochlorite for 10 min, washed twice with water, and stained. The pits developed blue to purple color.

The Expression of RANKL was measured with real-time fluorescent quantitative polymerase chain reaction.

EV administration causes inhibition of bone resorption in murine osteoclasts.

Example 4.3 - Composite material of the present disclosure

In further examples scaffold material was combined with EVs according to the disclosure to test the composite material according to the disclosure. The tests were performed as described in examples 4.1 to 4.3 above, but with the composite material according to the disclosure.

It was found that osteoblast differentiation was synergistically enhanced, osteoclast activity was synergistically decreased and wound healing (measured by fibroblast scratch assay) was synergistically improved (cf. also figures 6 & 9 and the description thereof), as compared to the assays where the cells were only treated with EVs of this disclosure.

Example 4.4 - Enzyme-linked immunosorbent assay (ELISA) of cell culture supernatants

Example 4.4.1 - Mouse Osteoprotegerin - ELISA

Protein detection was performed using Mouse Osteoprotegerin (OPG)/TNFRSF11B Quantikine ELISA Kit (R&D Systems) according to the manufacturer's protocol. Secreted Osteoprotegerin levels were calculated by comparison to a standard curve at an absorbance of 450 nm (Infinite M Nano plate reader, Tecan Life Science). At least four independent experiments were performed, and each sample was measured in duplicates. Values were normalized to respective controls.

Example 4.4.2 - Mouse Alkaline phosphatase - ELISA

Protein detection was performed using Mouse Alkaline Phosphatase (ALP)/ ab285274 - ELISA Kit (Abcam) according to the manufacturer's protocol. Secreted Alkaline Phosphatase levels were calculated by comparison to a standard curve at an absorbance of 450 nm (Infinite M Nano plate reader, Tecan Life Science). At least three independent experiments were performed, and each sample was measured in duplicates. Values were normalized to respective controls.

*Example 4.4.3 - Human TNFRSF11 B (OPG) - ELISA*

Protein detection was performed using Human TNFRSF1 1B (OPG) - ELISA Kit (Invitrogen) according to the manufacturer's protocol. Secreted human Osteoprotegerin levels were calculated by comparison to a standard curve at an absorbance of 450 nm (Infinite M Nano plate reader, Tecan Life Science). At least three independent experiments were performed, and each sample was measured in duplicates. Values were normalized to respective controls.

*Example 4.4.4 - Multiplex immunoassay*

Protein detection was performed using Mouse ProcartaPlex Mix & Match 8-Plex (Invitrogen) according to the manufacturer's protocol. In the multiplex immunoassay we targeted IL-1$\beta$, IL-10, IL-33, IL-6, M-CSF, MIP2$\alpha$ (CXCL2), RANKL and TNF$\alpha$. Secreted target levels were analysed using Bio-Plex (Bio-Rad). Concentrations were determined by comparing values to a standard curve. The experiment was performed three times.

Example 4.5 - Gene expression analysis

Osteoblasts were differentiated for 6 days and stimulated with hPLEV-F and TNF$\alpha$ for 14 to 24 hours. For the analysis of osteoclasts, primary murine bone marrow cells were seeded in M-CSF-containing medium as described before. After 24h hPLEV-F and after 48h TNF$\alpha$-stimulus were applied before ending the experiment one day later.

RNA was isolated with TRIzol Reagent (Thermo Fisher Scientific)/1-bromo-3-chloropropane on day 10 for mOB and on day 5 for osteoclasts. Purification was performed using RNA Clean and Concentrator-5 kit (Zymo Research) according to the manufacturer's instructions. The concentration and purity of RNA were measured with Nanodrop 2000 (Avantor). RNA was transcribed to cDNA with SuperScript IV Reverse Transcriptase (Thermo Fisher Scientific) according to the manufacturer's protocol. For quantitative RTPCR gene expression levels, Luminaris HiGreen Master Mix (Thermo Fisher Scientific) was used and monitored by qTOWER3 (Analytic JENA). Gapdh (glyceraldehyde 3-phosphate dehydrogenase) and Rps29 (40S ribosomal protein S29) served as respective reference genes. Sequences of all primers are depicted in Table 1. Each primer pair was tested with template dilution series for the calculation of efficiency followed by the analysis of melting curve and agarose gel electrophoresis to exclude primer dimers. Analysis of data was performed using the $\Delta\Delta$CT-method.

**Table 1.** Murine qRT-PCR-Primer used in analysis of primary osteoblasts and osteoclasts

| Gene | Gene Symbol | NCBI Gene ID | Primer Sequence (5'-3' direction) |
|---|---|---|---|
| Osteocalcin/bone gamma-carboxyglutamate protein 2 (Bglap2) | Ocn | 12097 | Fw  CAGACAAGTCCCACACAGCA (SEQ ID No. 1) |
| | | | Rev CTTGG-CATCTGTGAGGTCAG (SEQ ID No. 2) |
| Osteoprotegerin/ Tnfrsf11b tumor necrosis factor receptor superfamily, member 11b | Opg | 18383 | Fw  CCGAGTGTGTGAG-TGTGAGG (SEQ ID No. 3) |
| | | | Rev TGTGTTTCGCTCTGGGGTTC (SEQ ID No. 4) |
| Tumor necrosis factor receptor superfamily, member 11a (Activator of NF-kB) | Tnfrsf11 a/ Rank | 21934 | Fw  TGCAGCTCAACAAGGATACG (SEQ ID No. 5) |
| | | | Rev GTGCAGTTGGTCCAAGGTTT (SEQ ID No. 6) |
| Tumor necrosis factor (ligand) | Rankl | 21943 | Fw |

| | | | |
|---|---|---|---|
| superfamily, member 11 (Tnfsf11)/ Receptor Activator of NF-kB-Ligand | | | GCAGAAGGAACTGCAACACA (SEQ ID No. 7) |
| | | | Rev GATGGTGAGGTGTGCAAATG (SEQ ID No. 8) |
| Runt-related transcription factor 2 | Runx2 | 12393 | Fw GCCGGGAATGATGAGAAC (SEQ ID No. 9) |
| | | | Rev GGACCGTCCACTGTCACTT (SEQ ID No. 10) |
| Tartrate-resistant-acid-phosphatase 5 | Trap | 11433 | Fw CCAATGCCAAAGAGATCGCC (SEQ ID No. 11) |
| | | | Rev TCTGTGCAGAGACGTTGCCAAG (SEQ ID No. 12) |
| Collagen type I alpha 1 chain | Col1A1 | 12842 | Fw GAGAGCATGACCGATGGATT (SEQ ID No. 13) |
| | | | Rev TGAGCTCGATCTCGTTGGAT (SEQ ID No. 14) |
| Tumor necrosis factor alpha | Tnf-α | 51926 | Fw ACGGCATGGATCTCAAAGAC (SEQ ID No. 15) |
| | | | Rev GTGGGTGAGGAGCACGTAGT (SEQ ID No. 16) |
| Glyceraldehyde-3-phospate dehydrogenase | Gapdh | 14433 | Fw TGTGAACGGATTTGGCCGTA (SEQ ID No. 17) |

| | | | Rev ACTGTGCCGTTGAATTTGCC (SEQ ID No. 18) |
|---|---|---|---|
| 40S ribosomal protein S29 | Rps29 | 6235 | Fw  GAAGTTCGGCCAGGGTTCC (SEQ ID No. 19) |
| | | | Rev GAAGCCTATGTCCTTCGCGT (SEQ ID No. 20) |

Example 4.6 - Phosphoproteomic analysis

*Example 4.6.1 - Sample preparation*

For the phosphoproteomic analysis mOB were seeded at $1.0 \times 10^5$ in a 6-well plate in proliferation medium. After 48h of incubation at 37°C, medium was changed and hPLEV-F were added. The TNF$\alpha$ stimulus was applied 30min prior to ending the experiment on day 4. Cells were washed twice with a washing buffer (PBS+1% protease/phosphatase inhibitor (Halt™ Protease Inhibitor Cocktail, Thermo Fisher Scientific) +1% EDTA) at 4°C and then detached from the well in 500$\mu$l washing buffer. The suspension was then centrifuged at $1000 \times g$ for 10 min at 4°C. Everything but the cellular components was removed and the pellet frozen at -80°C.

After thawing, lysis buffer was added to the samples to a final concentration of 5% SDS, 100 mM HEPES and 50 mM DTT. The samples were sonicated (Bioruptor Plus) for 10 cycles (30 sec ON/60 sec OFF) at a high setting at 20°C, followed by boiling at 95°C for 7 min. Reduction was followed by alkylation with iodoacetamide (final concentration 15 mM) for 30 min at room temperature in the dark. Samples were acidified with phosphoric acid (final concentration 2.5%), and seven times the sample volume of S-trap binding buffer was added (100 mM TEAB, 90% methanol). Samples were bound on 96-well S-trap micro plate (Protifi) and washed three times with binding buffer. Trypsin in 50 mM TEAB pH 8.5 was added to the samples (1 $\mu$g per sample) and incubated for 1 h at 47°C. The samples were eluted in three steps with 50 mM TEAB pH 8.5, elution buffer 1 (0.2% formic acid in water) and elution buffer 2 (50% acetonitrile and 0.2% formic acid). Before phosphopeptide enrichment, samples were filled up to 210 $\mu$l. Phosphorylated peptides were enriched using Fe(III)-IMAC cartridges (Agilent) in an automated fashion using the standard protocol from the AssayMAP Bravo Platform (Agilent Technologies). In short, Fe(III)-IMAC cartridges were first primed with 100 $\mu$l of priming buffer (100% ACN, 0.1% TFA) and equilibrated with 50 $\mu$L of OASIS elution buffer. After loading the samples into the cartridge, the cartridges were washed with OASIS elution buffer, while the syringes were washed with priming buffer. The phosphopeptides were eluted with 25 $\mu$L of 1% ammonia directly into 25 $\mu$L of 10% FA. Samples were dried down with a speed vacuum centrifuge and stored at -20 °C until LC-MS analysis. For whole proteome analysis, the flow through after phosphoenrichment was dried down with a speed vacuum centrifuge and stored at -20 °C until LC-MS analysis.

*Example 4.6.2 - LC-MS Data independent analysis (DIA)*

For whole cell proteomics on Evosep, samples were loaded on Evotips (Evosep) according to the manufacturer's instructions. In short, Evotips were first washed with Evosep buffer B (acetonitrile, 0.1% formic acid), conditioned with 100% isopropanol and equilibrated with Evosep buffer A. Afterwards, the samples were loaded on the Evotips and washed with Evosep buffer A. The loaded Evotips were topped up with buffer A and placed in the machine. Peptides were separated using the Evosep One system (Evosep) equipped with a 15 cm $\times$ 150 $\mu$m i.d. packed with a 1.5 $\mu$m Reprosil-Pur C18 bead column (Evosep Performance, EV-1137, PepSep) heated at 45°C with a butterfly sleeve oven (Phoenix S&T). The samples were run with a preprogrammed proprietary Evosep gradient of 44 min (30 samples per day) using water and 0.1% formic acid and solvent B acetonitrile and 0.1% formic acid as solvents. The LC was coupled to an Orbitrap Exploris 480 (Thermo Fisher Scientific) using PepSep Sprayers and a Proxeon nanospray source. The peptides were introduced into the mass spectrometer via a PepSep Emitter 150-$\mu$m outer diameter $\times$ 10-$\mu$m inner diameter, heated at 300°C, and a spray voltage of 2 kV was applied. The injection capillary temperature was set at 300°C. The radio frequency ion funnel was set to 30%. For DIA data acquisition, full scan mass spectrometry (MS) spectra with a mass range of 350-1650 m/z were acquired in profile mode in the Orbitrap with a resolution of 120,000 FWHM. The default charge state was set to 2+, and the filling time was set at a maximum of 20 ms with a limitation of $3 \times 10^6$ ions. DIA scans were acquired with 40 mass window segments of differing widths across the MS1 mass range. Higher collisional dissociation fragmentation (normalized collision energy 30%) was applied, and MS/MS spectra were acquired with a resolution of 30,000 FWHM with a fixed first mass of 200 m/z after accumulation of $1 \times 106$ ions or after filling time of 45 ms (whichever occurred first). Data were acquired in profile mode. For data acquisition and processing of the raw data, Xcalibur 4.4 (Thermo) and Tune version 4.0 were used.

For phosphopeptides, samples were reconstituted in in MS Buffer (5% acetonitrile, 95% Milli-Q water, with 0.1% formic acid) and spiked with iRT peptides (Biognosys). Peptides were separated in trap/elute mode using the nanoAcquity Ultra-High Performance Liquid Chromatography system (Waters, Waters Corporation) equipped with a trapping (Waters nanoEase M/Z Symmetry C18, 5$\mu$m, 180 $\mu$m $\times$ 20 mm) and an analytical column (Waters nanoEase M/Z C18 HSS T3, 1.7$\mu$m, 75$\mu$m $\times$ 250mm). Solvent A was water and 0.1% formic acid, and solvent B was acetonitrile and 0.1% formic acid. 5 $\mu$l of the sample were loaded with a constant flow of solvent A at 5 $\mu$l/min onto the trapping column. Trapping time was 6 min. Peptides were eluted via the analytical column with a constant flow of 0.3 $\mu$l/min. During the elution step, the percentage of solvent B increased in a nonlinear fashion from 0-40% in 60 min. Total run time was 75 min. The LC was coupled to an Orbitrap Fusion Lumos (Thermo Fisher Scientific) using the Proxeon nanospray source. The peptides were introduced into the mass spectrometer via a Pepsep emitter with integrated liquid junction150-$\mu$m outer diameter $\times$ 20-$\mu$m inner diameter, 2.6 cm length (Bruker) heated at 300 °C, and a spray voltage of 2 kV was applied. The capillary temperature was set at 300°C. The radio frequency ion funnel was set to 30%. Full scan mass spectrometry (MS) spectra with mass range 350-1650 m/z were acquired in profile mode in the Orbitrap with resolution of 120,000 FWHM. The default charge state was set to 3+. The filling time was set at maximum of 60 ms with limitation of $3 \times 106$ ions. DIA scans were acquired with 40 mass window segments of differing widths across the MS1 mass range. Higher collisional dissociation fragmentation (stepped normalized collision energy; 25, 27.5, and 30%) was applied and MS/MS spectra were acquired with a resolution of 30,000 FWHM with a fixed first mass of 200 m/z after accumulation of $3 \times 106$ ions or after filling time of 35 ms (whichever occurred first). Data were acquired in profile mode. For data acquisition and processing of the raw data Xcalibur 4.5 (Thermo) and Tune version 4.0 were used.

*Example 4.6.3 - Data analysis*

DIA raw data were analyzed using the directDIA pipeline in Spectronaut (v.18, Biognosysis). The data were searched against a species specific (Mus Musculus, 16.747 entries) and a contaminants (247 entries) Swissprot database.

For whole proteome analysis, the data were searched with the following variable modifications: Oxidation (M) and Acetyl (Protein N-term). A maximum of 2 missed cleavages for trypsin and 5 variable modifications were allowed. The identifications were filtered to satisfy FDR of 1 % on peptide and protein level. Relative quantification was performed in Spectronaut for each paired comparison using the replicate samples from each condition. The data (candidate table) and data reports (protein quantities) were then exported and further data analysed and visualization were performed with R-studio using in-house pipelines and scripts. To select significant proteins, a log2FC cutoff of 0.58 and a q-value <0.05 were defined.

For phosphoproteomics analysis, the data were searched with the following modifications: Carbamidomethyl (C) (Fixed) and Oxidation (M), Acetyl (Protein N-term), Phospho (STY) (Variable). PTM localization probability was set to 0.75 and consolidation of phosphosites was sum based. A maximum of 2 missed cleavages for trypsin and 5 variable modifications were allowed. The identifications were filtered to satisfy FDR of 1 % on peptide and protein level. Relative quantification at phosphosite level was performed in Spectronaut for each paired comparison using the replicate samples from each condition. The data (candidate table) and data reports (protein quantities) were then exported and further data analyses and visualization were performed with Rstudio using in-house pipelines and scripts. To select significant phosphosites, a log2FC cutoff of 0.58 and a qvalue<0.05 were defined. For identifying the most regulated kinases and the corresponding regulated top phosphosites, a modified version of PhosR was used. Signalling pathways were analysed by the Over-Representation Analysis (ORA) method using the WEB-based GEne SeT AnaLysis Toolkit available at http://www.webgestalt.org/ (Liao, Wang, Jaehnig, Shi, & Zhang, 2019), with GENEOntology Biological Process, GENEOntology Cellular Compartment, KEGG and Reactome as functional databases.

*Example 4.6.3 - Bone augmentation matrix*

For mimicking a 3D bone microenvironment, a resorbable ceramic-collagen foam with incorporated the $\beta$-TCP-collagen composite of the disclosure (Cerasorb® Mouldable foam, Curasan AG) was used. It is composed of 85 wt-% $\beta$-tricalcium phosphate ($\beta$-TCP) particles, ranging from 150 to 2000 $\mu$m in diameter, and 15 wt-% porcine collagen, which includes 80 wt-% type I collagen, 15 wt-% elastin, and 5 wt-% type III collagen, with a porosity of 65 wt-% (H. Zheng et al., 2014). The foam was cut into 0.6cm $\times$ 0.6 cm $\times$ 0.2cm cubes and cells were seeded in a density of $1,4 \times 10^4$ cells/well in a 24 well plate with 600$\mu$l proliferation medium (as described above), covering the foam completely. After two days, the medium was exchanged for a differentiation medium as described above. After days 6, 9, 12 and 15 medium was changed.

On day 7, supernatants were collected for analysis of protein secretion. On day 17, samples were fixed with 4 wt-% PFA for 15min and washed for 2 cycles with PBS.

*Example 4.6.4 - Immunofluorescence staining of bone cells within the bone substitute matrix*

Fixed samples were processed overnight in a Histokinette (Tissue processor TP1020, Leica) and embedded (Histocore Arcadia modular tissue embedding system, Leica) in paraffine (Histosec® Pastilles, Merck). After 48h of freezing, samples were cut using a Microtome (Rotary Microtome RM2265, Leica)- preparing cuts of 9 $\mu$m and placing (Paraffine Stretch Bath 1052, GFL) them on slides (SuperFrost® PLUS Microscope Slides, VWR).

For cellular osteocalcin staining, slides were prepared using a deparaffinization series (2x 10min xylol, 2x10min 100% ethanol, 2x 5min 96% ethanol, 2x 5min 70% ethanol, 5min $H_2O$). After deparaffinization, slides were washed 2x with PBS and preconditioned using 1,5% normal-goat serum (NGS) + 0.1% TX-100 (Triton). Then an anti-osteocalcin-antibody (rabbit ab93876, Abcam, 1:500 in 1,5% NGS) was applied overnight at 4°C. After 3 washing cycles using PBS, the secondary antibody was applied (Alexa Fluor 488 anti-rabbit IgG (H+L), Invitrogen, 1:1,000 in 1.5% NGS). Thereafter, nuclei were counterstained using a 4',6-diamidino-2-phenylindole, dihydrochloride (DAPI) core staining (Thermo Fisher Scientific) 1:10,000 in PBS. After washing the excess dye using 3x PBS and 1x $ddH_2O$, slides were covered with coverslips and Mowiol (Mowiol 4-88, Carl Roth).

Example 4.7 - Evaluation of osteocalcin immunofluorescence

Imaging for the evaluation of osteocalcin activation and immunofluorescent staining was performed with the inverted confocal laser scanning microscope TCS SP5 (Leica). Slides were scanned in multiple layers and then organized in z-stacks. To analyse the number of DAPI-positive cells, the Fiji software (https://imagej.net/Fiji; version number 1.54g, accessed on 26 January 2024) was used. Microscopic imaging of each experiment was performed under identical settings for each treatment condition (ctrl and hPLEV-F-treated).

Example 4.8 - Isolation and cultivation of human osteoblasts

Collection of human oral bone was approved by the Institutional Review Board of the University Hospital Jena (No. 2019-1440-Material) and carried out according to The Code of Ethics of the World Medical Association (Declaration of Helsinki). All patients had signed informed consent.

For human osteoblast cultures, bone tissue was collected during dental implant procedures during the subsequent drilling steps. Bone pieces were stored in sterile PBS. First, soft tissue was removed using a scalpel followed by two subsequent washing steps with PBS. If necessary, bone was cut in small pieces prior to mechanical deterioration by three 10 s vortex-steps. In between, bone fragments were allowed to settle down before PBS was exchanged. After the final washing step, tissue underwent collagenase digestion (375 U/ml Collagenase II in DMEM) for 2 h at 37°C. Afterwards, bone fragments were washed three times in proliferation medium (DMEM low glucose (Thermo-Fisher Scientific), 100 U/ml penicillin, 100 $\mu$g/ml streptomycin (Thermo-Fisher Scientific), 50 $\mu$g/ml Gentamycin (Sigma-Aldrich), 1.25 $\mu$g/ml Amphotericin B (Sigma-Aldrich)) and transferred to a T25-flask with 5 ml proliferation medium. Medium was changed for the first time after 5 days of incubation. Subsequent media changes were performed three times a week until cells reached confluency. For infection and differentiation experiments, cells were seeded in a density $1.4 \times 10^4$ cells per well on 24-well-plates. After two days in culture, proliferation medium was exchanged for differentiation medium (proliferation medium supplemented with 100 $\mu$g/ml L-ascorbic acid, 10 nM $\beta$-glycerol phosphate and 100 nM dexamethasone). To analyse osteoblast differentiation, cells were incubated in differentiation media for up to 14 days. Cell culture medium was changed every 2 to 3 days.

Example 4.9 - Statistical analyses

Pairwise comparisons were performed with two-tailed Student's t-test. Differences between more than two groups were assessed using two-way ANOVA and post hoc test (Tukey). All values are presented as mean $\pm$ SE. Differences between groups were considered statistically significant at $P < 0.05$. Analyses were performed in Graph-Pad/InStat3 (GraphPad Software Inc.). For exclusion of single data points, Grubb's outlier test was performed.

**Example 5. 3D-Printing**

In general, the printing paste can be prepared following this protocol:

1. Preparing the Paste

a. Dissolving collagen:

- Dilute 0.5 M acetic acid to 0.05 M (1:10)

  ◦ 4 ml of 0.5 M acetic acid + 36 ml sterile water

- Weigh collagen using a precision scale:

  ◦ Tear small pieces using tweezers
  ◦ It is relatively stable and can be torn like cotton

- Gradually transfer collagen into a 50 ml Falcon tube

  ◦ Pack it down to fit the collagen, as 1 g is very voluminous

- Then, pipette 20 ml of the 0.05 M acetic acid onto it

  ◦ Swirl (invert and place back)
  ◦ If needed, distribute collagen using tweezers iii. The collagen dissolves slowly, becoming gel-like

- Add the remaining 20 ml of 0.05 M acetic acid

  ◦ Swirl again

- To fully dissolve, store overnight at 4°C in the refrigerator

  ◦ Sealed in a sterile manner (lid + parafilm)

b.) Mixing collagen with inorganic material:

  ◦ Weigh the granules according to the composition 85:15 w%
  ◦ Fold the granules into the mixture

c.) Optionally add EVs of the present disclosure dissolved in buffer (e.g. PBS).

2. Storing the Paste

- Store at 4°C

3. Using the paste in the screen printing process.

**Example 5.1. Manufacturing of printing pastes and inks for three-dimensional printing of bone replacement material including extracellular vesicles.**

An amount of 1 g collagen is solubilized in 40 ml 0.05 M acetic acid at 4°C over night. The obtained bioink is further supplemented with beta-tri-calcium-phosphate (beta-TCP) 85:15 w%. Furthermore, 10 ml of an EV/PBS suspension (extracellular vesicles in phosphate buffered saline) with an EV concentration of $2.5 \times 10^9$/ml is added.
The ink is stored at 4°C till further use.

**Example 5.2. Manufacture of three-dimensional biocompatible matrix**

The ink composition for 3D printing is used to produce a three-dimensional bone replacement product in a 3D screen printing unit. The ink from example 1 is pre-heated or pre-cooled to a temperature of 4-50°C, preferably 20°C. The screen printing squeegees are used with an angle of 50°-80°, preferably 65° and a hardness of 65-85 Shore for flooding, preferably 75 Shore, and 40-90 Shore for printing, preferably 55 Shore. The screen mesh is composed of polyamide, polyester, or steel with 8-400 threads/cm, preferably 48 threads/cm, and a thread thickness of 27-300 μm, preferably 55 μm. The screen EOM ("Emulsion over Mesh") can vary between 8-150 μm, preferably 20-25 μm. The printing process to build-up the geometry in z-axis is a continuous repetition of flooding, printing, and curing. The ink is flooded and printed through the screen mesh with the squeegees at a temperature between 4-65°C, preferably 20-40°C. Humidity in the printing chamber during the printing process is adjusted to 20-100%, preferably 56%. The curing of the printed ink is executed at 0-180°C using IR, convection drying, UV, or a cooling system. The increment of the printed layers is 40 μm. Preferably, the bioink from example 1 are cured at temperatures between 4-40°C. Humidity in the curing chamber can be adjusted to 10-60%, preferably 40%. The printing process is repeated until the final size of the three-dimensional product is achieved. The printing process can involve a combination and/or rotation of the ink. Furthermore, the printing process can involve several screens with different mesh compositions and geometries. When inks are used, the active ingredient, i.e. extracellular vesicles derived from animal cells, preferably derived from mammalian cells, more preferably derived from human cells, and artificially constructed lipid vesicles becomes incorporated into the three-dimensional biocompatible matrix by and during the printing process itself.

Alternatively, the extracellular vesicles are placed onto the printed bone replacement material. The step of applying such component can be performed by any suitable technology, including spraying, soaking, coating, imbibing, impregnating, applying and subsequently freeze-drying, nanosol technology, applying and subsequently exposing the printed bone replacement material to negative pressure or vacuum, etc..

**Claims**

1. Platelet-derived extracellular vesicle suitable for use as part of a bone replacement and/or bone rebuilding material, **characterized by** being positive for at least one biomarker selected from CD9, CD63, Syntenin, and TSG101, as well as combinations thereof.

2. The platelet-derived extracellular vesicle according to claim 1, wherein the platelet-derived extracellular vesicle is **characterized by** being additionally positive for at least one biomarker selected from the group consisting of CD41b, CD42a, CD42b, CD61, CD62P, von-Willebrand-factor, PDGF-BB, RANTES, and a protein of the ESCRT-complex, as well as a combination thereof, and/or being additionally positive for a growth factor selected from the group consisting of latent-transforming growth factor beta-binding protein, hepatocyte growth factor-like protein, and insulin-like growth factor-binding protein, as well as a combination thereof.

3. Composite material suitable for use as bone replacement and/or bone rebuilding material, **characterized in** comprising the platelet-derived extracellular vesicles according to any of claims 1 or 2 and further comprising at least one inorganic component and at least one organic component.

4. The composite material according to claim 3, wherein the inorganic component, is selected from the group consisting of calcium phosphate; sodium-, potassium-, calcium- and/or silicate-containing acid; neutral and/or alkaline bioglass; and glass ceramic, and a combination thereof; optionally in form of a granulate, the granulate optionally comprising biologically active, polygon-broken, rounded particles.

5. The composite material according to claim 4, wherein the calcium phosphate is selected from the group consisting of β-tricalcium phosphate, preferably β-tricalcium phosphate combined with 0 to 15% by weight of sodium magnesium silicate glass; monocalcium phosphate monohydrate; anhydrous monocalcium phosphate; dicalcium phosphate dihydrate; anhydrous dicalcium phosphate; tetra-calcium phosphate; whitlockite; octacalcium phosphate; hydroxyapatite; oxyapatite; type A carbonatapatite; type B carbonatapatite; calcium-deficient hydroxyapatite; amorphous calcium phosphate; amorphous carbonate-containing calcium phosphate; and calcium alkali orthophosphate glass ceramic, and a combination thereof.

6. The composite material according to any of claims 3 to 5, wherein the organic component is selected from the group consisting of a polymer, such as polypeptide, polysaccharide, and/or poly-lipid, and a combination thereof.

7. The composite material according to any of claims 3 to 6, wherein the organic component is selected from the group consisting of gelatine, collagen, glycosaminoglycan, hyaluronan, sodium hyaluronate, calcium hyaluronate, cellulose such as methylcellulose, ethylcellulose, propylcellulose, hydroxypropylcellulose, hydroxyethylcellulose, starch, dextran, hydroxyethyl starch, alginate, polyethylene glycol, albumin, and chitosan, or a combination thereof.

8. The composite material according to any of claims 3 to 7, wherein the organic component is selected from the group consisting of organically processed or demineralized allogeneic or xenogeneic bone material, synthetic polypeptide, parathyroid hormone, and osteogenic protein, or a combination thereof.

9. The composite material according to claim 8, wherein the osteogenic protein is selected from the group consisting of OP-1, OP-2, OP-3, BMP2, BMP3, BMP4, BMP5, BMP6, BMP-7, BMP9, BMP10, BMP11, BMP12, BMP14, BMP15, BMP16, IGF, TGF, PDGF, GDF1, GDF3, GDF5, GDF6, GDF7, GDF8, GDF9, GDF10, and GDF11, and a combination thereof.

10. The composite material according to claims 3 to 9, **characterized by** the inorganic component having at least one of the features selected from the group consisting of

 being a ceramic component comprising calcium, phosphate, $SiO_2$, $MgO$ and $Na_2O$,
 being a granulate whose particles have a $d_{50}$ value of at least 10 $\mu$m,
 being present in the composite material from 15 to 80 wt.-%, and
 having an interconnecting, open multi-porosity with micro-, meso- and macropores (pore diameter 0.05 $\mu$m - 500 $\mu$m) and a total porosity of from at least 35% up to 65%,
 as well as combinations thereof.

11. Use of the platelet-derived extracellular vesicle according to any of claims 1 or 2, or the composite material according to any of claims 3 to 10, as a stimulator of osteoblast activity and/or inhibitor of osteoclast activity in vitro and/or in cosmetics.

12. Method for production of a composite comprising the steps of:

 a. adding at least one organic component to water to form a suspension;
 b. adding at least one inorganic component; and, optionally, a crosslinking agent; to the suspension of step a. and mixing the components;
 c. transferring the solution from step b. into a mold and then drying the mixture by lyophilization to form the composite material;
 d. adding at least one platelet-derived extracellular vesicle to water or buffer to obtain a suspension;
 e. contacting the composite material of step c. with the suspension of step d..

13. Method for production of a bone replacement materialcomprising the steps of:

 a) dissolving collagen in an acidic solution,
 b.) mixing the dissolved collagen with the inorganic material according to claims 4 or 5 selected from the group consisting of calcium phosphate, sodium-, potassium-, calcium- and/or silicate-containing acid, neutral and/or alkaline bioglass, and glass ceramic, and a combination thereof; wherein the granulate comprises biologically active, polygon-broken, rounded particles and,
 c.) Optionally, adding the EVs according to claims 1 or 2suspended in buffer solution.
 b.) Optionally, storing the paste at 4°C until use;
 c.) Using the paste in a 3D screen printing unit in order to three-dimensional print a bone re-placement material.

14. The platelet-derived extracellular vesicle according to any of claims 1 or 2, or the composite material according to any of claims 3 to 10 for the use as a medicament, such as for use in the treatment of a disease or condition where bone replacement or bone rebuilding is desired, for example selected from the group consisting of broken bones, bone fractures, bone loss, bone degradation, dental soft tissue degradation, dental bone diseases, periondontitis, alveolar bone resorption, dental implant placement, jaw bone infections associated with bone loss, osteomyelitis, osteonecrosis of the jaw (ONJ), osteoporosis, osteopenia, Paget's disease of bone, osteomalacia, rickets, hyperparathyroidism when associated with bone reduction, osteogenesis imperfecta (brittle bone disease), multiple myeloma when associated with bone reduction, metastatic bone disease (bone metastases), avascular necrosis (osteonecrosis), fibrous dysplasia, Gaucher's disease, hyperthyroidism when associated with bone reduction, renal osteodystrophy, anorexia nervosa when associated with bone reduction, Cushing's syndrome, rheumatoid arthritis when associated with bone reduction, psoriatic arthritis when associated with bone reduction, sarcoidosis when associated with bone reduction, systemic lupus erythematosus (SLE), thalassemia, hypophosphatasia when associated with bone reduction, Camurati-Engel-mann disease, sickle cell disease when associated with bone reduction, chronic kidney disease when associated with bone reduction, marfan syndrome, Ehlers-Danlos syndrome (subtypes which are when associated with bone reduction), steroid-induced osteoporosis (resulting from long-term use of corticosteroids), inflammatory bowel disease (e.g., Crohn's disease, ulcerative colitis, which affect bone density), HIV/AIDS when associated with bone reduction, hemochromatosis when associated with bone reduction, post-polio syndrome affecting bones, diseases of dysfunction of bone metabolism, stimulation of osteoblasts, reduction of osteoclasts, migration/proliferation of (gingival) fibroblasts, and mastocytosis when associated with bone reduction, or combinations thereof.

**15.** Kit comprising the platelet-derived extracellular vesicle according to any of claims 1 or 2, and/or the composite material according to any of claims 3 - 10, further comprising at least one item selected from the group consisting of an applicator, a measuring cup, a mixing tool, a spatula, an instruction manual, a package leaflet, and a syringe, as well as any combination thereof.

FIGURE 1

FIGURE 2

FIGURE 3

FIGURE 4

FIGURE 5

FIGURE 6

FIGURE 7

**FIGURE 8**

FIGURE 9

|  | ctrl/ctrl | ctrl/EV | TNFα/ctrl | TNFα/EV |
|---|---|---|---|---|
| ctrl/ctrl |  | ns | * | ns |
| ctrl/EV | ns |  | * | ns |
| TNFα/ctrl | * | * |  | ** |
| TNFα/EV | ns | ns | ** |  |

FIGURE 10

FIGURE 11

FIGURE 12

FIGURE 13

FIGURE 13 (continued)

FIGURE 13 (continued)

FIGURE 13 (continued)

FIGURE 13 (continued)

**I**

**J**

FIGURE 13 (continued)

FIGURE 14

FIGURE 14 (continued)

FIGURE 14 (continued)

FIGURE 14 (continued)

FIGURE 14 (continued)

**K**

**L**

FIGURE 14 (continued)

FIGURE 14 (continued)

FIGURE 15

FIGURE 16

FIGURE 17

FIGURE 17 (continued)

FIGURE 17 (continued)

FIGURE 18

FIGURE 19

**FIGURE 19 (continued)**

Europäisches Patentamt

European Patent Office

Office européen des brevets

## EUROPEAN SEARCH REPORT

**Application Number**

EP 24 20 6692

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XU CHENYUE ET AL: "Platelet-Derived Exosomes Alleviate Knee Osteoarthritis by Attenuating Cartilage Degeneration and Subchondral Bone Loss", AMERICAN JOURNAL OF SPORTS MEDICINE., 8 August 2023 (2023-08-08), pages 2975-2985, XP093260613, WALTHAM, MA. ISSN: 0363-5465, DOI: 10.1177/03635465231188122 Retrieved from the Internet: URL:http://journals.sagepub.com/doi/full-xml/10.1177/03635465231188122 [retrieved on 2025-03-18] * abstract * * paragraph "Identificationof Plt-exos"; page 2976 * * paragraph "Plt-exos attenuated subchondral bone loss in vivo"; page 2979 * | 1,2,14, 15 | INV. A61L27/12 A61L27/36 A61L27/46 A61L27/54 |
| X | Antich-Rosselló M ET AL: "Platelet-derived extracellular vesicles promote osteoinduction of mesenchymal stromal cells", , 1 January 2020 (2020-01-01), pages 667-674, XP093260615, DOI: 10.1302/2046-3758.910.BJR- Retrieved from the Internet: URL:https://boneandjoint.org.uk/article/10.1302/2046-3758.910.BJR-2020-0111.R2 [retrieved on 2025-03-18] | 1-15 | TECHNICAL FIELDS SEARCHED (IPC) A61L |
| Y | * abstract * * bridging paragraph; page 671 - page 672 * * page 673, column 2, lines 1-9 * | 3-10,12, 13 | |

-----

-/--

The present search report has been drawn up for all claims

| Place of search | Date of completion of the search | Examiner |
|---|---|---|
| The Hague | 31 March 2025 | Guazzelli, Giuditta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

        .......................................................................................
& : member of the same patent family, corresponding document

EPO FORM 1503 03.82 (P04C01)

Europäisches Patentamt

European Patent Office

Office européen des brevets

## EUROPEAN SEARCH REPORT

**Application Number**

EP 24 20 6692

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LIU ANQI ET AL: "Optimized BMSC-derived osteoinductive exosomes immobilized in hierarchical scaffold via lyophilization for bone repair through Bmpr2/Acvr2b competitive receptor-activated Smad pathway", BIOMATERIALS, vol. 272, 1 May 2021 (2021-05-01), page 120718, XP093260871, AMSTERDAM, NL ISSN: 0142-9612, DOI: 10.1016/j.biomaterials.2021.120718 * abstract * * pages 2-3, paragraph 2.2 * * page 5, paragraph 2.4 * | 3-10,12, 13 | |
| Y | LIU LI ET AL: "Bone Marrow Mesenchymal Stem Cell-Derived Small Extracellular Vesicles Promote Periodontal Regeneration", TISSUE ENGINEERING PART A, vol. 27, no. 13-14, 1 July 2021 (2021-07-01), pages 962-976, XP093260872, ISSN: 1937-3341, DOI: 10.1089/ten.tea.2020.0141 * abstract * * paragraph "Preparation and detection of the BMSC-sEV nanocomposite hydrogels"; page 964 * | 3-10,12, 13 | TECHNICAL FIELDS SEARCHED (IPC) |

The present search report has been drawn up for all claims

| Place of search | Date of completion of the search | Examiner |
|---|---|---|
| The Hague | 31 March 2025 | Guazzelli, Giuditta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

    .......................................................................................
& : member of the same patent family, corresponding document

EPO FORM 1503 03.82 (P04C01)

**REFERENCES CITED IN THE DESCRIPTION**

**Patent documents cited in the description**

- US 8501170 B2